# EUROPEAN PATENT APPLICATION

(11) **EP 4 393 936 A1**
(43) Date of publication of application: **03.07.2024**
(21) Application number: 22860419.5
(22) Date of filing: 22.08.2022
(51) Int. Cl.: C07H 21/02, C07H 1/00, C12N 15/11, C12N 15/10, A61K 31/7088, A61P 37/02

(54) **CAPPED ANALOG AND USE THEREOF**

(30) Priority: 27.08.2021 CN 202110995875
(71) Applicant: Shanghai Hongene Biotech Corporation, Shanghai 201108 (CN); Shanghai Hongene Bioengineering Co., Ltd, Shanghai 201417 (CN)
(72) Inventor: CHEN, Li, Shanghai 201108 (CN); WEI, Minzhi, Shanghai 201108 (CN)
(74) Representative: V.O.
(86) International application number: PCT/CN2022/113813
(87) International publication number: WO 2023/025073

(57) **Abstract**

The present invention discloses a cap analog and use thereof. The structure of said cap analog is as shown in formula (I):

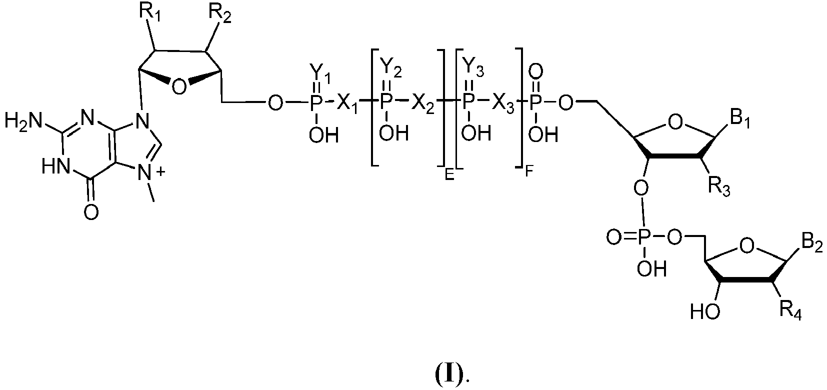

## Description

### TECHNICAL FIELD

The present invention relates to the field of chemistry and bio-engineering. In particular, the present invention relates to a serial of cap analogs for *in vitro* transcription (IVT) synthesis of mRNA and use of the cap analogs in mRNA synthesis.

### BACKGROUND ART

*In vitro* synthesis of mRNA has become an important tool for exogenous gene introduction and protein expression, and is widely used in the treatment and prevention of diseases. Immune responses induced by exogenous mRNA affects the expression of target proteins via various pathways.

N1-2'O-methylation is essential for blocking RIG-I activation, and knockdown of intracellular N1-2'O-transmethylase also causes immuno-stimulation of endogenous RNA. The combination of 2'-O-methylation and m⁷G capping produces a synergistic effect in reducing the binding affinity of RNA to RIG-I.

During mRNA transcription, IFIT1 and translation factor EIF4E competitively bind to mRNA template. Endogenous and exogenous mRNAs with cap structure and 2'-O-methylation are bound by EIF4E to initiate transcription. However, if an exogenous mRNA lacks 2'-O-methylation on the first ribose, the mRNA is recognized by IFIT1 which inhibits the binding of intracellular factors and blocks its transcription. N1 methylation alone may not be sufficient in protecting all the endogenous mRNAs from IFIT1, and the recognition of IFIT1 to 2'-O methylation on N1 and/or N2 ribose suggests that the methyl group conflicts with these protein residues.

Hence, there is an urgent need in the art to develop a new class of cap analogs to allow mRNAs synthesized by *in vitro* transcription to effectively inhibit the onset of immune response.

### CONTENTS OF THE INVENTION

It is an objection of the present invention to provide a class of new cap analogs of structural formula (I) and use thereof.

In a first aspect, the present invention provides a cap analog of formula (I):

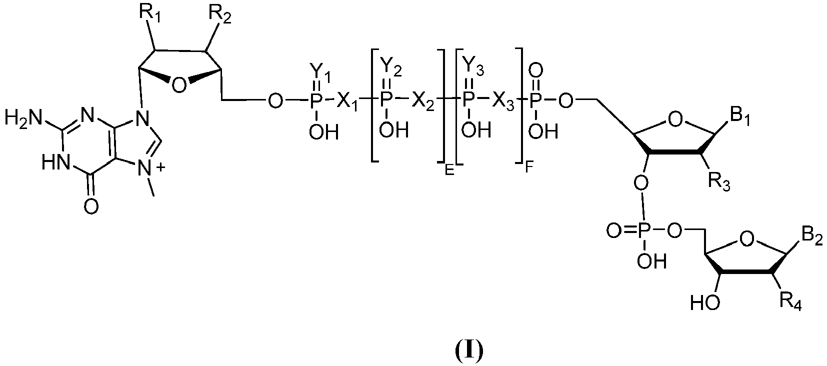

Wherein, in the formula, B₁ and B₂ are independently natural or modified base;
E and F are independently 0 or 1;
R₁ is H, OH, alkyl, O-alkyl, halo, or O, wherein said O taken together with carbon atoms at 3' position and 5' position form a bridged ring;
R₂ is H, OH, alkyl, O-alkyl, or halo;
R₃ is O-R₅-R₆;
R₄ is H, OH, O-methyl or O-R₅-R₆;
R₅ is substituted or unsubstituted C₁₋₂₀ alkyl;
R₆ is substituted or unsubstituted O-alkyl, substituted or unsubstituted S-alkyl, substituted or unsubstituted NH-alkyl, substituted or unsubstituted N-dialkyl, substituted or unsubstituted O-aryl, substituted or unsubstituted S-aryl, substituted or unsubstituted NH-aryl, substituted or unsubstituted O-aralkyl, substituted or unsubstituted S-aralkyl, substituted or unsubstituted NH-aralkyl, or H (when R₅ is substituted or unsubstituted C₂₋₂₀ alkyl);
X₁, X₂, and X₃ are independently O, CH₂, or NH;
Y₁, Y₂, and Y₃ are independently O, S, Se, or BH₃.

In some embodiments, R₃ is OCH₂CH₃, OCH₂OCH₃, or OCH₂CH₂OCH₃.

In some embodiments, R₄ is OH, OCH₂CH₃, OCH₂OCH₃, or OCH₂CH₂OCH₃.

In some embodiments, B₁ and B₂ are independently adenine, N6-methyladenine, guanine, uracil, or thymine.

In some embodiments, said cap analog is selected from: ^{m7}GpppA_{2'O-ethyl}pG, ^{m7}GpppA_{2'O-ethyl}pA, ^{m7}GpppA_{2'O-ethyl}pC, ^{m7}GpppA_{2'O-ethyl}pU, ^{m7}GpppC_{2'O-ethyl}pA, ^{m7}GpppC_{2'O-ethyl}pG, ^{m7}GpppC_{2'O-ethyl}pC, ^{m7}GpppC_{2'O-ethyl}pU, ^{m7}GpppG_{2'O-ethyl}pA, ^{m7}GpppG_{2'O-ethyl}pC, ^{m7}GpppG_{2'O-ethyl}pG, ^{m7}GpppG_{2'O-ethyl}pU, ^{m7}GpppU_{2'O-ethyl}pA, ^{m7}GpppU_{2'O-ethyl}pC, ^{m7}GpppU_{2'O-ethyl}pC, or ^{m7}GpppU_{2'O-ethyl}pU.

In some embodiments, said cap analog is selected from: ^{m7}G_{3'Ome}pppA_{2'O-ethyl}pG, ^{m7}G_{3'Ome} pppA_{2'O-ethyl}pA, ^{m7}G_{3'Ome} pppA_{2'O-ethyl}pC, ^{m7}G_{3'Ome}pppA_{2'O-ethyl}pU, ^{m7}G_{3'Ome}pppC_{2'O-ethyl}pA, ^{m7}G_{3'Ome}pppC_{2'O-ethyl}pG, ^{m7}G_{3'Ome}pppC_{2'O-ethyl}pC, ^{m7}G_{3'Ome}pppC_{2'O-ethyl}pU, ^{m7}G_{3'Ome}pppG_{2'O-ethyl}pA, ^{m7}G_{3'Ome}pppG_{2'O-ethyl}pC, ^{m7}G_{3'Ome}pppG_{2'O-ethyl}pG, ^{m7}G_{3'Ome}pppG_{2'O-ethyl}pU, ^{m7}G_{3'Ome}pppU_{2'O-ethyl}pA, ^{m7}G_{3'Ome}pppU_{2'O-ethyl}pC, ^{m7}G_{3'Ome}pppU_{2'O-ethyl}pC, or ^{m7}G_{3'Ome}pppU_{2'O-ethyl}pU.

In some embodiments, said cap analog is selected from: ^{m7}G_{2'Ome}pppA_{2'O-ethyl}pG, ^{m7}G_{2'Ome}pppA_{2'O-ethyl}pA, ^{m7}G_{2'Ome}pppA_{2'O-ethyl}pC, ^{m7}G_{2'Ome}pppA_{2'O-ethyl}pU, ^{m7}G_{2'Ome}pppC_{2'O-ethyl}pA, ^{m7}G_{2'Ome}pppC_{2'O-ethyl}pG, ^{m7}G_{2'Ome}pppC_{2'O-ethyl}pC, ^{m7}G_{2'Ome}pppC_{2'O-ethyl}pU, ^{m7}G_{2'Ome}pppG_{2'O-ethyl}pA, ^{m7}G_{2'Ome}pppG_{2'O-ethyl}pC, ^{m7}G_{2'Ome}pppG_{2'O-ethyl}pG, ^{m7}G_{2'Ome}pppG_{2'O-ethyl}pU, ^{m7}G_{2'Ome}pppU_{2'O-ethyl}pA, ^{m7}G_{2'Ome}pppU_{2'O-ethyl}pC, ^{m7}G_{2'Ome}pppU_{2'O-ethyl}pC, or ^{m7}G_{2'Ome}pppU_{2'O-ethyl}pU.

In some embodiments, said cap analog is selected from: ^{m7}Gppp(N6-methyladenine)_{2'O-ethyl}pG, ^{m7}Gppp(N6-methyladenine)_{2'O-ethyl}pA, ^{m7}Gppp(N6-methyladenine)_{2'O-ethyl}pC, ^{m7}Gppp(N6-methyladenine)_{2'O-ethyl}pU, ^{m7}G_{2'Ome}ppp(N6-methyladenine)_{2'O-ethyl}pA, ^{m7}G_{2'Ome}ppp(N6-methyladenine)_{2'O-ethyl}pG, ^{m7}G_{2'Ome}ppp(N6-methyladenine)_{2'O-ethyl}pC, ^{m7}G_{2'Ome}ppp(N6-methyladenine)_{2'O-ethyl}pU, ^{m7}G_{3'Ome}ppp(N6-methyladenine)_{2'O-ethyl}pA, ^{m7}G_{3'Ome}ppp(N6-methyladenine)_{2'O-ethyl}pC, ^{m7}G_{3'Ome}ppp(N6-methyladenine)_{2'O-ethyl}pC, or ^{m7}G_{3'Ome}ppp(N6-methyladenine)_{2'O-ethyl}pU.

In some embodiments, said cap analog is selected from: ^{m7}GpppA_{2'O-MOE}pG, ^{m7}GpppA_{2'O-MOE}pA, ^{m7}GpppA_{2'O-MOE}pC, ^{m7}GpppA_{2'O-MOE}pU, ^{m7}GpppC_{2'O-MOE}pA, ^{m7}GpppC_{2'O-MOE}pG, ^{m7}GpppC_{2'O-MOE}pC, ^{m7}GpppC_{2'O-MOE}pU, ^{m7}GpppG_{2'O-MOE}pA, ^{m7}GpppG_{2'O-MOE}pC, ^{m7}GpppG_{2'O-MOE}pG, ^{m7}GpppG_{2'O-MOE}pU, ^{m7}GpppU_{2'O-MOE}pA, ^{m7}GpppU_{2'O-MOE}pC, ^{m7}GpppU_{2'O-MOE}pC, or ^{m7}GpppU_{2'O-MOE}pU.

In some embodiments, said cap analog is selected from: ^{m7}G_{3'Ome}pppA_{2'O-MOE}pG, ^{m7}G_{3'Ome} pppA_{2'O-MOE}pA, ^{m7}G_{3'Ome}pppA_{2'O-MOE}pC, ^{m7}G_{3'Ome}pppA_{2'O-MOE}pU, ^{m7}G_{3'Ome}pppC_{2'O-MOE}pA, ^{m7}G_{3'Ome}pppC_{2'O-MOE}pG, ^{m7}G_{3'Ome}pppC_{2'O-MOE}pC, ^{m7}G_{3'Ome}pppC_{2'O-MOE}pU, ^{m7}G_{3'Ome}pppG_{2O-'MOE}pA, ^{m7}G_{3'Ome}pppG_{2'O-MOE}pC, ^{m7}G_{3'Ome}pppG_{2'O-MOE}pG, ^{m7}G_{3'Ome}pppG_{2'O-MOE}pU, ^{m7}G_{3'Ome}pppU_{2'O-MOE}pA, ^{m7}G_{3'Ome}pppU_{2'O-MOE}pC, ^{m7}G_{3'Ome}pppU_{2'O-MOE}pC, or ^{m7}G_{3'Ome}pppU_{2'O-MOE}pU.

In some embodiments, said cap analog is selected from: ^{m7}G_{2'Ome}pppA_{2'O-MOE}pG, ^{m7}G_{2'Ome} pppA_{2'O-MOE}pA, ^{m7}G_{2'Ome} pppA_{2'O-MOE}pC, ^{m7}G_{2'Ome}pppA_{2'O-MOE}pU, ^{m7}G_{2'Ome}pppC_{2'O-MOE}pA, ^{m7}G_{2'Ome}pppC_{2'O-MOE}pG, ^{m7}G_{2'Ome}pppC_{2'O-MOE}pC, ^{m7}G_{2'Ome}pppC_{2'O-MOE}pU, ^{m7}G_{2'Ome}pppG_{2'O-MOE}pA, ^{m7}G_{2'Ome}pppG_{2'O-MOE}pC, ^{m7}G_{2'Ome}pppG_{2'O-MOE}pG, ^{m7}G_{2'Ome}pppG_{2'O-MOE}pU, ^{m7}G_{2'Ome}pppU_{2'O-MOE}pA, ^{m7}G_{2'Ome}pppU_{2'O-MOE}pC, ^{m7}G_{2'Ome}pppU_{2'O-MOE}pC, or ^{m7}G_{2'Ome}pppU_{2'O-MOE}pU.

In some embodiments, said cap analog is selected from: ^{m7}Gppp(N6-methyladenine)_{2'O-MOE}pG, ^{m7}Gppp(N6-methyladenine) _{2'O-MOE}pA, ^{m7}Gppp(N6-methyladenine)_{2'O-MOE}pC, ^{m7}Gppp(N6-methyladenine) _{2'O-MOE}pU, ^{m7}G_{2'Ome}ppp(N6-methyladenine)_{2'O-MOE}pA, ^{m7}G_{2'Ome}ppp(N6-methyladenine) _{2'O-MOE}pG, ^{m7}G_{2'Ome}ppp(N6-methyladenine)_{2'O-MOE}pC, ^{m7}G_{2'Ome}ppp(N6-methyladenine)_{2'O-MOE}pU, ^{m7}G_{3'Ome}ppp(N6-methyladenine)_{2'O-MOE}pA, ^{m7}G_{3'Ome}ppp(N6-methyladenine)_{2'O-MOE}pC, ^{m7}G_{3'Ome}ppp(N6-methyladenine) _{2'O-MOE}pC, or ^{m7}G_{3'Ome}ppp(N6-methyladenine) _{2'O-MOE}pU.

In some embodiments, the structure of said cap analog is as shown in Formula (I-a) or (I-b):

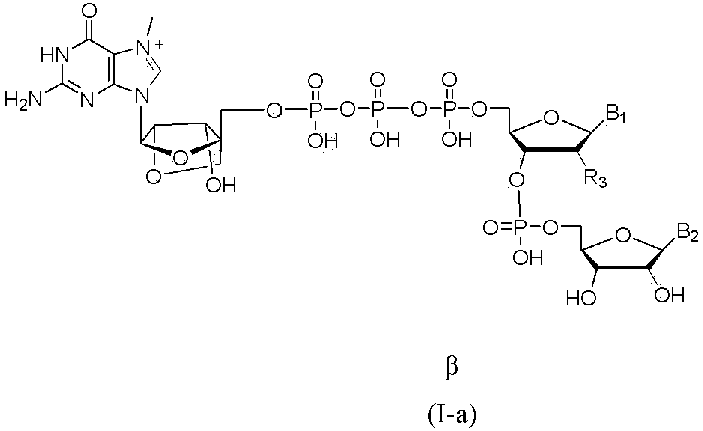

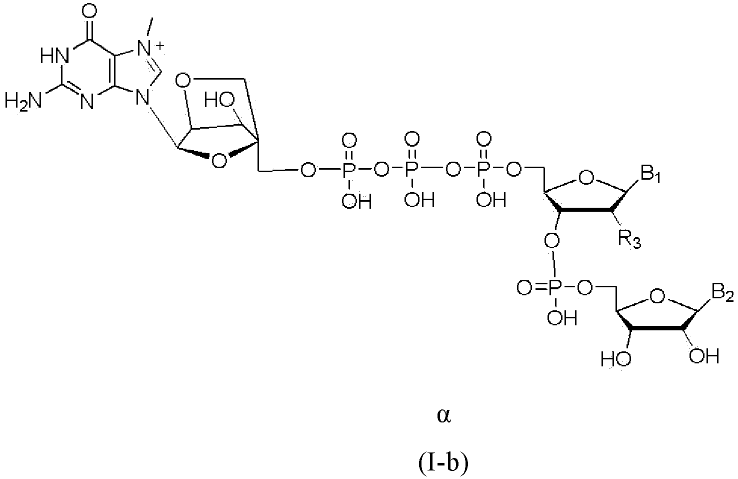

Wherein, in the formula, B₁ and B₂ are independently natural or modified base; and
R₃ is O-R₅-R₆.

In some embodiments, R₃ is OCH₂CH₃, OCH₂OCH₃, or OCH₂CH₂OCH₃.

In some embodiments, B₁ and B₂ are independently adenine, N6-methyladenine, guanine, uracil, or thymine.

In a second aspect, the present invention provides a polynucleotide encoding a polypeptide of interest, wherein said polynucleotide comprises:
(a) at least one ORF region;
(b) at least one Kozak sequence of 5'UTR;
(c) 3'UTR; and
(d) at least one cap analog as described above initially capped at the 5' end.

In a third aspect, the present invention provides a pharmaceutical composition comprising the aforementioned polynucleotide of the present invention and a pharmaceutically acceptable carrier.

In some embodiments, said carrier is selected from: lipid nanoparticles (LNPs), liposomes, polymeric nanoparticles, solid lipid nanoparticles, or emulsions.

In a fourth aspect, the present invention provides a method for preparing the aforementioned polynucleotide of the present invention comprising:
(1) preparing a DNA template;
(2) producing the aforementioned polynucleotide of the present invention via *in vitro* transcription, wherein the reaction system comprises RNA polymerase, nucleoside triphosphate, and the aforementioned cap analog of the present invention.

In some embodiments, said nucleoside triphosphate may be a nucleoside triphosphate of natural base or a modified nucleoside triphosphate, or a non-natural nucleoside triphosphate; preferably ATP, CTP, GTP, UTP, 5me-CTP, 5me-UTP, PseudoUTP, or N1-me-PseudoUTP.

In some embodiments, RNA polymerase is a phage-derived RNA polymerase.

In some embodiments, said RNA polymerase is selected from: T7, SP6, or T3.

In some embodiments, said RNA polymerase is a mutant of a phage-derived RNA polymerase, which has at least 80%, 90%, 95%, 96%, 97%, 98%, 99% amino acid identity to a natural sequence.

Accordingly, the present invention provides a new class of cap analogs, by which the onset of immune response can be effectively inhibited by mRNAs synthesized by *in vitro* transcription.

### DESCRIPTOIN OF THE DRAWINGS

**Figs. 1-3** show the synthesis pathway for the cap analogs of Example 1.
**Fig. 4** shows protein expression levels of mRNAs with various cap analogs prepared according to the Examples.
**Fig. 5** shows relative binding ability of mRNAs with different cap analogs prepared according to the Examples to RIG-I.
**Fig. 6** shows relative binding ability of mRNAs with different cap analogs prepared according to the Examples to IFIT1.
**Fig. 7** shows results of immunogenicity induced by the mRNAs with different cap analogs prepared according to the Examples.
**Fig. 8** shows protein expression levels of mRNAs with cap analog ^{m7}G_{Beta-D-LNA}pppA_{2'O-MOE}pG and ^{m7}G_{Alpha-L-LNA}pppA_{2'O-ethyl} pG prepared according to the Examples.
**Fig. 9** shows relative binding ability of mRNAs with different cap analogs ^{m7}G_{Beta-D-LNA}pppA_{2'O-MOE}pG and ^{m7}G_{Alpha-L-LNA}pppA_{2'O}-_{ethyl}pG prepared according to the Examples to RIG-I.
**Fig. 10** shows relative binding ability of mRNAs with different cap analogs ^{m7}G_{Beta-D-LNA}pppA_{2'O-MOE}pG and ^{m7}G_{Alpha-L-LNA}pppA_{2'O-ethyl}pG prepared according to the Examples to IFIT1.
**Fig. 11** shows results of immunogenicity induced by mRNAs with different cap analogs ^{m7}G_{Beta-D-LNA}pppA_{2'O-MOE}pG and ^{m7}G_{Alpha-L-LNA}pppA_{2'O-eth}yₗ pG prepared according to the Examples.
**Fig. 12** shows HPLC chromatograms for the capping efficiency of different cap nucleotides prepared according to the Examples; wherein,

A shows the chromatogram of ^{m7}GpppApG, B shows the chromatogram of ^{m7}G pppA_{2'Ome} pG, C shows the chromatogram of ^{m7}G_{3'Ome}pppA_{2'O-ethyl}pG, D shows the chromatogram of ^{m7}G_{2'Ome}pppA_{2'O-ethyl}pG, E shows the chromatogram of ^{m7}G_{3'Ome}pppA_{2'O-MOE}pG, F shows the chromatogram of ^{m7}G_{2'Ome}pppA_{2'O-MOE}pG, G shows the chromatogram of ^{m7}Gppp(N6-methyladenine)_{2'O-MOE}pG, H shows the chromatogram of ^{m7}G_{2'Ome}pppC_{2'O-ethyl}pC, and I shows the chromatogram of ^{m7}G_{3'Ome}pppA_{2'O-MOE}pU.

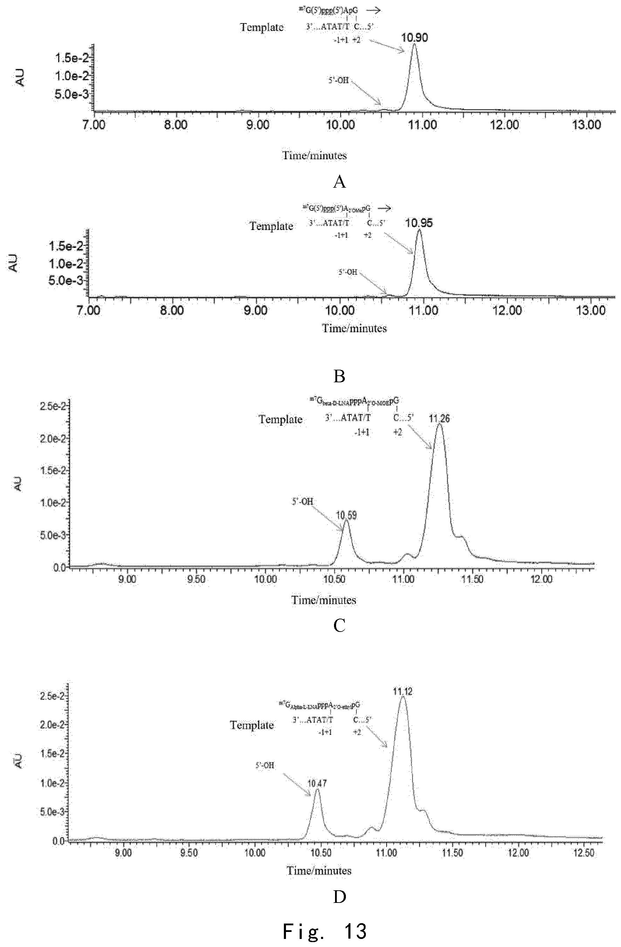

Fig. 13 shows HPLC chromatograms for the capping efficiency of different cap nucleotides prepared according to the Examples; wherein,

A shows the chromatogram of ^{m7}GpppApG, B shows the chromatogram of ^{m7}G pppA_{2'Ome} pG (control), C shows the chromatogram of ^{m7}G_{Beta-D-LNA}pppA_{2'O-MOE}pG, and D shows the chromatogram of ^{m7}G_{Alpha-L-LNA}pppA_{2'O-ethyl} pG.

### PARTICULAR EMBODIMENTS

According to extensive and thorough researches, the inventors have synthesized a serial of tri-oligonucleic acid polymers, and discovered for the first time that by producing mRNAs using cap analogs of formula (I) via IVT, the binding of the mRNAs to IFIT1 and the inhibition for the activation of RIG-I can be hindered, such that the onset of immune reactions can be strictly and effectively inhibited. On the above basis, the present invention was completed.

The present invention provides a cap analog of formula (I):

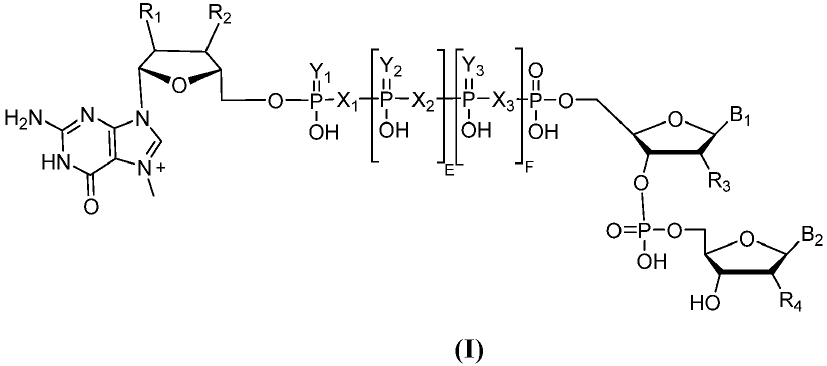

Wherein, in the formula, B₁ and B₂ are independently natural or modified bases;
E and F are independently 0 or 1;
R₁ is H, OH, alkyl, O-alkyl (for example, but not limited to, O-methyl), halo, or O, wherein said O taken together with carbon atoms at 3' position and 5' position form a bridged ring;
R₂ is H, OH, alkyl, O-alkyl (for example, but not limited to, O-methyl), or halo;
R₃ is O-R₅-R₆;
R₄ is H, OH, O-methyl or O-R₅-R₆;
R₅ is substituted or unsubstituted C₁₋₂₀ alkyl;
R₆ is substituted or unsubstituted O-alkyl, substituted or unsubstituted S-alkyl, substituted or unsubstituted NH-alkyl, substituted or unsubstituted N-dialkyl, substituted or unsubstituted O-aryl, substituted or unsubstituted S-aryl, substituted or unsubstituted NH-aryl, substituted or unsubstituted O-aralkyl, substituted or unsubstituted S-aralkyl, or substituted or unsubstituted NH-aralkyl;
when R₅ is substituted or unsubstituted C₂₋₂₀ alkyl, R₆ can be H;
X₁, X₂, and X₃ are independently O, CH₂, or NH;
Y₁, Y₂, and Y₃ are independently O, S, Se, or BH₃.

In some embodiments of the present invention, R₆ is H and R₅ is substituted or unsubstituted C₂₋₅ alkyl, said R₃ and R₄ are (but not limited to) independently O-ethyl, O-propyl, O-isopropyl, O-butyl, O-isobutyl, O-t-butyl, O-pentyl, and so on; or R₄ is OH, R₃ is, but not limited to, O-ethyl, O-propyl, O-isopropyl, O-butyl, O-isobutyl, O-t-butyl, O-pentyl, and so on.

In some embodiments of the present invention, R₅ is substituted or unsubstituted C₁₋₅ alkyl and R₆ is substituted or unsubstituted C₁₋₅ alkyl, said R₃ and R₄ are independently, but not limited to, O-methylene-O-methyl, O-ethylene-O-methyl, O-propylene-O-methyl, O-butylene-O-methyl, O-pentylene-O-methyl, O-methylene-O-ethyl, O-methylene-O-propyl, O-methylene-O-isopropyl, O-methylene-O-butyl, O-methylene-O-isobutyl, O-methylene-O-t-butyl, O-methylene-O-pentyl, O-ethylene-O-ethyl, O-ethylene-O-propyl, O-ethylene-O-isopropyl, O-ethylene-O-butyl, O-ethylene-O-isobutyl, O-ethylene-O-t-butyl, O-ethylene-O-pentyl, O-propylene-O-ethyl, O-propylene-O-propyl, O-propylene-O-isopropyl, O-propylene-O-butyl, O-propylene-O-isobutyl, O-propylene-O-t-butyl, O-propylene-O-pentyl, O-butylene-O-ethyl, O-butylene-O-propyl, O-butylene-O-isopropyl, O-butylene-O-butyl, O-butylene-O-isobutyl, O-butylene-O-t-butyl, O-butylene-O-pentyl, O-pentylene-O-ethyl, O-pentylene-O-propyl, O-pentylene-O-isopropyl, O-pentylene-O-butyl, O-pentylene-O-isobutyl, O-pentylene-O-t-butyl, O-pentylene-O-pentyl, and so on; or R₄ is OH, R₃ is, but not limited to, O-methylene-O-methyl, O-ethylene-O-methyl, O-propylene-O-methyl, O-butylene-O-methyl, O-pentylene-O-methyl, O-methylene-O-ethyl, O-methylene-O-propyl, O-methylene-O-isopropyl, O-methylene-O-butyl, O-methylene-O-isobutyl, O-methylene-O-t-butyl, O-methylene-O-pentyl, O-ethylene-O-ethyl, O-ethylene-O-propyl, O-ethylene-O-isopropyl, O-ethylene-O-butyl, O-ethylene-O-isobutyl, O-ethylene-O-t-butyl, O-ethylene-O-pentyl, O-propylene-O-ethyl, O-propylene-O-propyl, O-propylene-O-isopropyl, O-propylene-O-butyl, O-propylene-O-isobutyl, O-propylene-O-t-butyl, O-propylene-O-pentyl, O-butylene-O-ethyl, O-butylene-O-propyl, O-butylene-O-isopropyl, O-butylene-O-butyl, O-butylene-O-isobutyl, O-butylene-O-t-butyl, O-butylene-O-pentyl, O-pentylene-O-ethyl, O-pentylene-O-propyl, O-pentylene-O-isopropyl, O-pentylene-O-butyl, O-pentylene-O-isobutyl, O-pentylene-O-t-butyl, O-pentylene-O-pentyl, and so on.

Exemplary tri-oligonucleic acid polymers of the cap analogs of formula (I) of the present invention are shown in the table below:

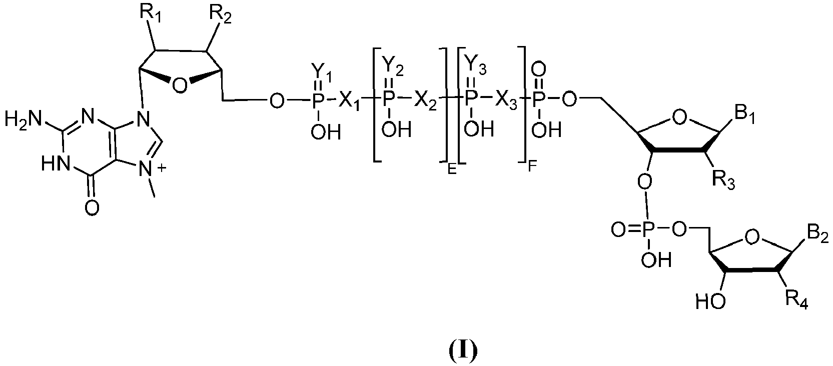

| **Structure** | **Description of the Structure** |
|---|---|
| ^{m7}GpppA_{2'O-ethyl}pG, ^{m7}GpppA_{2'O-ethyl}pA, ^{m7}GpppA_{2'O-ethyl}pC, ^{m7}GpppA_{2'O-ethyl}pU, ^{m7}GpppC_{2'O-ethyl}pA, ^{m7}GpppC_{2'O-ethyl}pG, ^{m7}GpppC_{2'O-ethyl}pC, ^{m7}GpppC_{2'O-ethyl}pU, ^{m7}GpppG_{2'O-ethyl}pA, ^{m7}GpppG_{2'O-ethyl}pC, ^{m7}GpppG_{2'O-ethyl}pG, ^{m7}GpppG_{2'O-ethyl}pU, ^{m7}GpppU_{2'O-ethyl}pA, ^{m7}GpppU_{2'O-eth}ylpC, ^{m7}GpppU_{2'O-ethyl}pC, ^{m7}GpppU_{2'O-ethyl}pU | In formula (I), R₁, R₂, and R₄ are all OH; E and F are independently 0 or 1; Y₁ and Y₂, or Y₃ are independently O, S, Se, or BH₃; R₃ is O-ethyl; B₁ and B₂ are independently natural base |
| ^{m7}G_{3'Ome}pppA_{2'O-eth}ylpG, ^{m7}G_{3'Ome}pppA_{2'O-ethyl}pA, ^{m7}G_{3'Ome}pppA_{2'O-ethyl}pC, ^{m7}G_{3'Ome}pppA_{2'O-ethyl}pU, ^{m7}G_{3'Ome}pppC_{2'O-ethyl}pA, ^{m7}G_{3'Ome}pppC_{2'O-eth}ylpG, ^{m7}G_{3'Ome}pppC_{2'O-ethyl}pC, ^{m7}G_{3'Ome}pppC_{2'O-ethyl}pU, ^{m7}G_{3'Ome}pppG_{2'O-ethyl}pA, ^{m7}G_{3'Ome}pppG_{2'O-ethyl}pC, ^{m7}G_{3'Ome}pppG_{2'O-ethyl}pG, ^{m7}G_{3'Ome}pppG_{2'O-ethyl}pU, ^{m7}G_{3'Ome}pppU_{2'O-ethyl}pA, ^{m7}G_{3'Ome}pppU_{2'O-ethyl}pC, ^{m7}G_{3'Ome}pppU_{2'O-ethyl}pC, ^{m7}G_{3'Ome}pppU_{2'O-ethyl}pU | In formula (I), R₁ is O-methyl; R₂ and R₄ are both OH; E and F are independently 0 or 1; Y₁ and Y₂, or Y₃ are independently O, S, Se, or BH₃; R₃ is O-ethyl; B₁ and B₂ are independently natural base |
| ^{m7}G_{2'Ome}pppA_{2'O-ethyl}pG, ^{m7}G_{2'Ome}pppA_{2'O-ethyl}pA, ^{m7}G_{2'Ome}pppA_{2'O-ethyl}pC, ^{m7}G_{2'Ome}pppA_{2'O-ethyl}pU, ^{m7}G_{2'Ome}pppC_{2'O-ethyl}pA, ^{m7}G_{2'Ome}pppC_{2'O-ethyl}pG, ^{m7}G_{2'Ome}pppC_{2'O-ethyl}pC, ^{m7}G_{2'Ome}pppC_{2'O-ethyl}pU, ^{m7}G_{2'Ome}pppG_{2'O-ethyl}pA, ^{m7}G_{2'Ome}pppG_{2'O-ethyl}pC, ^{m7}G_{2'Ome}pppG_{2'O-ethyl}pG, ^{m7}G_{2'Ome}pppG_{2'O-ethyl}pU, ^{m7}G_{2'Ome}pppU_{2'O-ethyl}pA, ^{m7}G_{2'Ome}pppU_{2'O-ethyl}pC, ^{m7}G_{2'Ome}pppU_{2'O-ethyl}pC, ^{m7}G_{2'Ome}pppU_{2'O-ethyl}pU | In formula (I), R₂ is O-methyl; R₁ and R₄ are both OH; E and F are independently 0 or 1; Y₁ and Y₂, or Y₃ are independently O, S, Se, or BH₃; R₃ is O-ethyl; B₁ and B₂ are independently natural base |
| ^{m7}Gppp(N6-methyladenine)_{2'O-ethyl}pG, ^{m7}Gppp(N6-methyladenine)_{2'O-ethyl}pA, ^{m7}Gppp(N6-methyladenine)_{2'O-ethyl}pC, ^{m7}Gppp(N6-methyladenine)_{2'O-ethyl}pU, ^{m7}G_{2'Ome}ppp(N6-methyladenine)_{2'O-ethyl}pA, ^{m7}G_{2'Ome}ppp(N6-methyladenine)_{2'O-ethyl}pG, ^{m7}G_{2'Ome}ppp(N6-methyladenine)_{2'O-ethyl}pC, ^{m7}G_{2'Ome}ppp(N6-methyladenine)_{2'O-ethyl}pU, ^{m7}G_{3'Ome}ppp(N6-methyladenine)_{2'O-ethyl}pA, ^{m7}G_{3'Ome}ppp(N6-methyladenine)_{2'O-ethyl}pC, ^{m7}G_{3'Ome}ppp(N6-methyladenine)_{2'O-ethyl}pC, ^{m7}G_{3'Ome}ppp(N6-methyladenine)_{2'O-ethyl}pU | In formula (I), R₁ and R₂ are independently O-methyl or OH; R₄ is OH; E and F are independently 0 or 1; Y₁ and Y₂, or Y₃ are independently O, S, Se, or BH₃; R₃ is O-ethyl; B₁ is N6-methyladenine ; B₂ is natural base |
| ^{m7}GpppA_{2'O-MOE}pG, ^{m7}GpppA_{2'O-MOE}pA, ^{m7}GpppA_{2'O-MOE}pC, ^{m7}GpppA_{2'O-MOE}pU, ^{m7}GpppC_{2'O-MOE}pA, ^{m7}GpppC_{2'O-MOE}pG, ^{m7}GpppC_{2'O-MOE}pC, ^{m7}GpppC_{2'O-MOE}pU, ^{m7}GpppG_{2'O-MOE}pA, ^{m7}GpppG_{2'O-MOE}pC, ^{m7}GpppG_{2'O-MOE}pG, ^{m7}GpppG_{2'O-MOE}pU, ^{m7}GpppU_{2'O-MOE}pA, ^{m7}GpppU_{2'O-MOE}pC, ^{m7}GpppU_{2'O-MOE}pC, ^{m7}GpppU_{2'O-MOE}pU | In formula (I), R₁, R₂ and R₄ are all OH; E and F are independently 0 or 1; Y₁ and Y₂, or Y₃ are independently O, S, Se, or BH₃; R₃ is O-methylene-O-ethyl; B₁ and B₂ are independently natural base |
| ^{m7}G_{3'Ome}pppA_{2'O-MOE}pG, ^{m7}G_{3'Ome}pppA_{2'O-MOE}pA, ^{m7}G_{3'Ome}pppA_{2'O-MOE}pC, ^{m7}G_{3'Ome}pppA_{2'O-MOE}pU, ^{m7}G_{3'Ome}pppC_{2'O-MOE}pA, ^{m7}G_{3'Ome}pppC_{2'O-MOE}pG, ^{m7}G_{3'Ome}pppC_{2'O-MOE}pC, ^{m7}G_{3'Ome}pppC_{2'O-MOE}pU, ^{m7}G_{3'Ome}pppG_{2'O-'MOE}pA, ^{m7}G_{3'Ome}pppG_{2'O-MOE}pC, ^{m7}G_{3'Ome}pppG_{2'O-MOE}pG, ^{m7}G_{3'Ome}pppG_{2'O-MOE}pU, ^{m7}G_{3'Ome}pppU_{2'O-MOE}pA, ^{m7}G_{3'Ome}pppU_{2'O-MOE}pC, ^{m7}G_{3'Ome}pppU_{2'O-MOE}pC, ^{m7}G_{3'Ome}pppU_{2'O-MOE}pU | In formula (I), R₁ is O-methyl; R₂ and R₄ are both OH; E and F are independently 0 or 1; Y₁ and Y₂, or Y₃ are independently O, S, Se, or BH₃; R₃ is O-methylene-O-ethyl; B₁ and B₂ are independently natural base |
| ^{m7}G_{2'Ome}pppA_{2'O-MOE}pG, ^{m7}G_{2'Ome}pppA_{2'O-MOE}pA, ^{m7}G_{2'Ome}pppA_{2'O-MOE}pC, ^{m7}G_{2'Ome}pppA_{2'O-MOE}pU, ^{m7}G_{2'Ome}pppC_{2'O-MOE}pA, ^{m7}G_{2'Ome}pppC_{2'O-MOE}pG, ^{m7}G_{2'Ome}pppC_{2'O-MOE}pC, ^{m7}G_{2'Ome}pppC_{2'O-MOE}pU, ^{m7}G_{2'Ome}pppG_{2'O-MOE}pA, ^{m7}G_{2'Ome}pppG_{2'O-MOE}pC, ^{m7}G_{2'Ome}pppG_{2'O-MOE}pG, ^{m7}G_{2'Ome}pppG_{2'O-MOE}pU, ^{m7}G_{2'Ome}pppU_{2'O-MOE}pA, ^{m7}G_{2'Ome}pppU_{2'O-MOE}pC, ^{m7}G_{2'Ome}pppU_{2'O-MOE}pC, ^{m7}G_{2'Ome}pppU_{2'O-MOE}pU | In formula (I), R₂ is O-methyl; R₁ and R₄ are both OH; E and F are independently 0 or 1; Y₁ and Y₂, or Y₃ are independently O, S, Se, or BH₃; R₃ is O-methylene- O-ethyl; B₁ and B₂ are independently natural base |
| ^{m7}Gppp(N6-methyladenine)_{2'O-MOE}pG, ^{m7}Gppp(N6-methyladenine)_{2'O-MOE}pA, ^{m7}Gppp(N6-methyladenine)_{2'O-MOE}pC, ^{m7}Gppp(N6-methyladenine)_{2'O-MOE}pU, ^{m7}G_{2'Ome}ppp(N6-methyladenine)_{2'O-MOE}pA, ^{m7}G_{2'Ome}ppp(N6-methyladenine)_{2'O-MOE}pG, ^{m7}G_{2'Ome}ppp(N6-methyladenine)_{2'O-MOE}pC, ^{m7}G_{2'Ome}ppp(N6-methyladenine)_{2'O-MOE}pU, ^{m7}G_{3'Ome}ppp(N6-methyladenine)_{2'O-MOE}pA, ^{m7}G_{3'Ome}ppp(N6-methyladenine)_{2'O-MOE}pC, ^{m7}G_{3'Ome}ppp(N6-methyladenine)_{2'O-MOE}pC, ^{m7}G_{3'Ome}ppp(N6-methyladenine)_{2'O-MOE}pU | In formula (I), R₁ and R₂ are independently O-methyl or OH; R₄ is OH; E and F are independently 0 or 1; Y₁ and Y₂, or Y₃ are independently O, S, Se, or BH₃; R₃ is O-methylene- O-ethyl; B₁ is N6-methyladenine ; B₂ is natural base |

In some embodiments, the present invention provides a cap analog having the structure of formula (I-a) or formula (I-b), wherein formula (I-a) and formula (I-b) are in different stereo-conformations, and wherein formula (I-a) is in β-conformation, and formula (I-b) is in α-conformation: 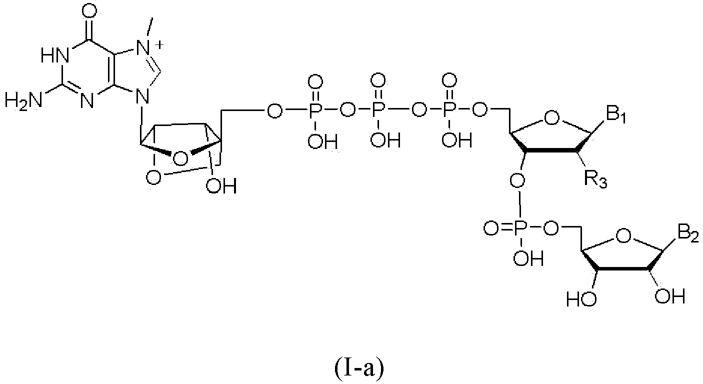 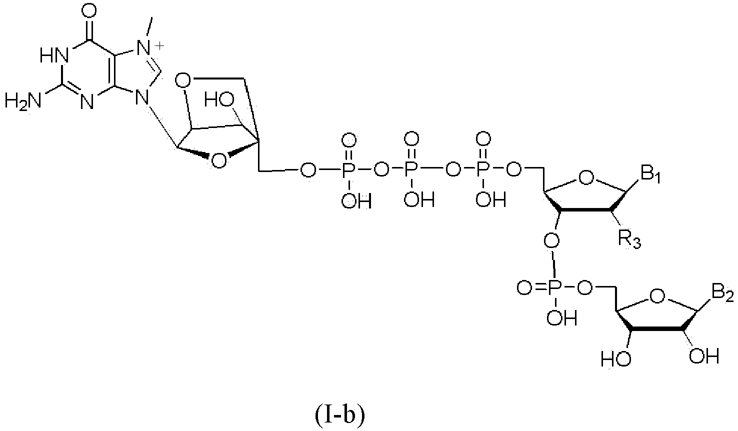
wherein, in the formulas, B₁ and B₂ are independently natural or modified bases;
R₃ is O-R₅-R₆;
R₅ is substituted or unsubstituted C₁₋₂₀ alkyl;
R₆ is substituted or unsubstituted O-alkyl, substituted or unsubstituted S-alkyl, substituted or unsubstituted NH-alkyl, substituted or unsubstituted N-dialkyl, substituted or unsubstituted O-aryl, substituted or unsubstituted S-aryl, substituted or unsubstituted NH-aryl, substituted or unsubstituted O-aralkyl, substituted or unsubstituted S-aralkyl, or substituted or unsubstituted NH-aralkyl;
when R₅ is substituted or unsubstituted C₂₋₂₀ alkyl, R₆ can be H.

In some embodiments of the present invention, in formula (I-a) or formula (I-b), R₆ is H and R₅ is substituted or unsubstituted C₂₋₅ alkyl, said R₃ is, but not limited to, O-ethyl, O-propyl, O-isopropyl, O-butyl, O-isobutyl, O-t-butyl, O-pentyl, and so on.

In some embodiments of the present invention, in formula (I-a) or formula (I-b), R₅ is substituted or unsubstituted C₁₋₅ alkyl and R₆ is substituted or unsubstituted C₁₋₅ alkyl, said R₃ is, but not limited to, O-methylene-O-methyl, O-ethylene-O-methyl, O-propylene-O-methyl, O-butylene-O-methyl, O-pentylene-O-methyl, O-methylene-O-ethyl, O-methylene-O-propyl, O-methylene-O-isopropyl, O-methylene-O-butyl, O-methylene-O-isobutyl, O-methylene-O-t-butyl, O-methylene-O-pentyl, O-ethylene-O-ethyl, O-ethylene-O-propyl, O-ethylene-O-isopropyl, O-ethylene-O-butyl, O-ethylene-O-isobutyl, O-ethylene-O-t-butyl, O-ethylene-O-pentyl, O-propylene-O-ethyl, O-propylene-O-propyl, O-propylene-O-isopropyl, O-propylene-O-butyl, O-propylene-O-isobutyl, O-propylene-O-t-butyl, O-propylene-O-pentyl, O-butylene-O-ethyl, O-butylene-O-propyl, O-butylene-O-isopropyl, O-butylene-O-butyl, O-butylene-O-isobutyl, O-butylene-O-t-butyl, O-butylene-O-pentyl, O-pentylene-O-ethyl, O-pentylene-O-propyl, O-pentylene-O-isopropyl, O-pentylene-O-butyl, O-pentylene-O-isobutyl, O-pentylene-O-t-butyl, O-pentylene-O-pentyl, and so on; or R₄ is OH, R₃ is, but not limited to, O-methylene-O-methyl, O-ethylene-O-methyl, O-propylene-O-methyl, O-butylene-O-methyl, O-pentylene-O-methyl, O-methylene-O-ethyl, O-methylene-O-propyl, O-methylene-O-isopropyl, O-methylene-O-butyl, O-methylene-O-isobutyl, O-methylene-O-t-butyl, O-methylene-O-pentyl, O-ethylene-O-ethyl, O-ethylene-O-propyl, O-ethylene-O-isopropyl, O-ethylene-O-butyl, O-ethylene-O-isobutyl, O-ethylene-O-t-butyl, O-ethylene-O-pentyl, O-propylene-O-ethyl, O-propylene-O-propyl, O-propylene-O-isopropyl, O-propylene-O-butyl, O-propylene-O-isobutyl, O-propylene-O-t-butyl, O-propylene-O-pentyl, O-butylene-O-ethyl, O-butylene-O-propyl, O-butylene-O-isopropyl, O-butylene-O-butyl, O-butylene-O-isobutyl, O-butylene-O-t-butyl, O-butylene-O-pentyl, O-pentylene-O-ethyl, O-pentylene-O-propyl, O-pentylene-O-isopropyl, O-pentylene-O-butyl, O-pentylene-O-isobutyl, O-pentylene-O-t-butyl, O-pentylene-O-pentyl, and so on.

Further provided in the present invention is a method for the preparation of a cap analog having the structure of formula (I), formula (I-a), or formula (I-b), for example, but not limited to, by solid phase synthesis of oligonucleotide, wherein the method comprises:
(1) preparing pNpN dinucleotide on solid phase synthesizer using raw materials 5'-O-DMT-2'-O-TBDMS phosphoramidite (rA^{Ac}, rC^{Ac}, rG^{dmf}, U), 2'-O-MOE-3'-O-phosphoramidite (A_{2'O-MOE}^{Ac}, C_{2'O-MOE}^{Ac}, G_{2'O-MOE}^{dmf} U_{2'O-MOE}) and 2'-O-Ethyl-3'-O-phosphoramidite (A_{2'O-ethyl} ^{Ac}, C_{2'O-ethyl} ^{Ac}, G_{2'O-ethyl} ^{dmf}, U_{2'O-ethyl}), Bis-cyanoethyl-N,N-diisopropyl CED phosphoramidite, and so on;
(2) reacting pNpN dinucleotide with phosphor-site imidazolium salt of 7me-2'-O-4'-C-Locked-Guanosine 5'-diphosphate (^{m7}G_{Beta-D-LNA}DPIm), or 2'-O,4'-C-methylene-alfa-L-ribofuranosyl Guanosine 5'-diphosphate (^{m7}G_{Alpha-L-LNA}DPIm), or N7-methylguanosine 5'-diphosphate (^{m7}GDPIm), to form a cap analog having the structure of formula (I), formula (I-a), or formula (I-b).

In some embodiments of the present invention, the method further comprises a step of purifying the thus obtained cap analog, for example, but not limited to, the purification using DEAE-650s (Toyopearl) and semi-preparation RT-HPLC column.

The present invention further provides a method for the *in vitro* transcription synthesis of mRNA using the cap analog having the structure of formula (I), formula (I-a), or formula (I-b), comprising the steps of:
Step 1, preparing template DNA;
Step 2, preparing mixed enzymes and reaction buffer;
Step 3, carrying out *in vitro* transcription reaction;
Step 4, carrying out dephosphorylation treatment;
Step 5, purifying the reaction solution.

In above mentioned Step 1, the template is usually formed of a linear plasmid which comprises a target sequence inserted in the multiple cloning site and a promoter for the corresponding polymerase upstream from the target sequence, such as T7, T3, or SP6 promoter. The plasmid needs to be linearized before use. DNA can be linearized via digestion using suitable restriction endonucleases conventionally known in the art, and then purified by, for example, but not limited to phenol chloroform treatment followed by ethanol precipitation.

Restriction endonucleases usually used for the linearization of plasmid should be able to produce blunt end or 5'-overhang (linearizing template with an enzyme capable of producing 3'-overhang leads to abnormal transcript).

In above mentioned Step 2, the mixed enzymes can be RNA polymerase, nuclease inhibitor, pyrophosphatase, and so on.

In above mentioned Step 2, the reaction buffer prepared comprises but not limited to, Tris-HCl (pH 7.9), MgCl₂, spermidine, dithiothreitol (DTT), Triton X-100.

In above mentioned Step 3, the cap analog provide in the present invention can be used in the *in vitro* transcription reaction.

In above mentioned Step 4, the reagents that can be used in the dephosphorylation treatment may comprise but not limited to, Antarctic TABS phosphatase, shrimp alkaline phosphatase, calf alkaline phosphatase, and so on.

In above mentioned Step 5, conventional methods in the art can be used for the purification, for example, but not limited to, HPLC, Lithium chloride precipitation, ammonium acetate precipitation, and so on.

The present invention further provides a polynucleotide encoding the polypeptide of interest comprising the cap analog of the present invention, and a pharmaceutical composition comprising the polynucleotide encoding the polypeptide of interest, and so on.

Unless otherwise indicated, the terms used in the Specification and Claims of the present application have the following meanings:
As used herein, the term "cap analog" refers to a structure at the 5' end of a mature mRNA formed by post-transcriptional modification in eukaryocyte, i.e., m⁷GPPPN structure, also called methylguanosine cap. Such a structure can prevent the degradation of mRNA at the 5' end, help RNA transcripts pass through the selective pores of nuclear membrane and enter cytoplasm, enhance translation, and help complete the entire cleavage process, and so on.

As used herein, the terms "base" and "natural base" are interchangeably used (also called nucleobase or nitrogenous base), and refer to nitrogenous compounds for forming nucleoside which is a component in nucleotide; it can be adenine (A), guanine (G), cytosine (C), uracil (U), or thymine(T).

"Modified base" refers to a substance in which one or more hydrogen atoms on a natural base are substituted, for example, but not limited to, N6-methyladenine, and so on.

"Alkyl" refers to an aliphatic hydrocarbon group. The alkyl portion may be a saturated alkyl group (that is, it does not contain any unsaturated units, such as carbon-carbon double bonds or carbon-carbon triple bonds) or the alkyl portion may be an unsaturated alkyl group (that is, it contains at least one unsaturated unit). The alkyl portion, whether saturated or unsaturated, may be branched or straight chain. There may be from 1 to 8 carbon atoms (when present, a numerical range, such as "1 to 8" refers to each integer in the given range, e.g., "1 to 8 carbon atoms" means that it may contain 1 carbon atom, 2 carbon atoms, 3 carbon atoms, etc., up to an alkyl group containing 8 carbon atoms, though the present definition also includes the occurrence of the term "alkyl" when no numerical range is specified). The alkyl group of the compounds described herein may be designated as "C₁ -C₆ alkyl" or similar designations. By way of example, "C₁ -C₆ alkyl" refers to an alkyl chain having one, two, three, four, five or six carbon atoms. Typical alkyl groups include, but are not limited to, methyl, ethyl, propyl, isopropyl, butyl, isobutyl, tert-butyl, pentyl, hexyl, and the like.

"Aralkyl" refers to -alkyl-aryl, wherein the meanings of alkyl and aryl are as defined herein.

"Halo" refers to F, Cl, Br, I.

"Alkoxy" group refers to "O-alkyl" group, in which the meaning of alkyl is as defined herein.

"Aromatic" refers to a planar ring having a delocalized π electron system comprising 4n+2 π electrons, wherein n is an integer. An aromatic ring may consist of five, six, seven, eight, nine, ten or more atoms. An aromatic ring can be optionally substituted. The term "aromatic" may cover carbocyclic aryl ("aryl" such as phenyl) or heterocyclic aryl (or "heteroaryl", or "aromatic heterocycle") (such as pyridine).

"Substitution/substituted" refers to the situation wherein a reference group may be substituted with one or more additional groups selected separately and independently from alkyl, cycloalkyl, aryl, heteroaryl, heteroaliphatic hydrocarbons, hydroxyl, alkoxy, alkylthio, arylthio, alkylsulfinyl, arylsulfinyl, alkylsulfonyl, arylsulfonyl, cyano, halo, carbonyl, thiocarbonyl, nitro, haloalkyl, fluoroalkyl and amino (including mono- and di- substituted amino groups and their protected derivatives). For example, the substituents are selected from halogen, CF₃, OH, CN, NO₂, SO₃H, SO₂NH₂, SO₂Me, NH₂, COOH, CONH₂, alkoxy, -N(CH₃)₂ and alkyl.

The term "pharmaceutically acceptable carrier" means one or more compatible solid or liquid fillers or gel substances which are suitable for human use and which must be of sufficient purity and sufficiently low toxicity. "Compatibility" herein refers to the ability of the components of the composition to be admixed with the compounds of the invention and with each other without appreciably reducing the efficacy of the compounds. Examples of pharmacologically acceptable excipients or carrier components are cellulose and derivatives thereof (e.g., sodium carboxymethylcellulose, sodium ethyl-cellulose, cellulose acetate, etc.), gelatin, talc, solid lubricants (e.g., stearic acid, magnesium stearate), calcium sulphate, vegetable oils (e.g., soya bean oil, sesame oil, peanut oil, olive oil, etc.), polyols (e.g., propylene glycol, glycerol, mannitol, sorbitol, etc.), emulsifiers (e.g., Tween^{®}), lubricants (e.g., sodium dodecyl sulphate), coloring agent, flavoring agent, stabilizer, antioxidant, preservative, pyrogen-free water, and the like.

The main advantage of the present invention lies in that the cap analog of the present invention can effectively inhibit the onset of immune response.

It should be understood that, within the scope of the present invention, each of the above-described technical features of the present invention and each of the technical features specifically described below (e.g., in the examples) may be combined with each other, thereby forming new or preferred technical solution(s). Due to the control of the length of the Specification, this will not be repeated herein.

Various specific aspects, properties, and advantages of the above compounds, methods, and pharmaceutical compositions will be set forth in detail in the following description to clarify the present invention. It should be understood herein that the following detailed description and examples describe specific embodiments and are for reference purposes only. After reading the description of the present invention, a person skilled in the art may make various changes or modifications to the present invention, which also fall within the scope of the application as defined herein.

The invention is explained more specifically in the following embodiments. It should be understood, however, that these examples are intended to illustrate the invention and are not intended to limit the scope of the invention in any way. Experimental methods for which specific conditions are not indicated in the following examples are generally in accordance with conventional conditions, or in accordance with conditions recommended by the manufacturer. Unless otherwise indicated, portions and percentages are weight portions and weight percentages. The organic solvents used in the reactions of the examples were treated by drying methods known in the art.

The **LC-MS analyses** involved in determining capping efficiency in the following examples are as follows:
Equipment: Waters UPLC H-Class
Column: ACQUITY UPLC Oligonucleotide BEH C18, 2.1*150mm
Column temperature: 75 °C
Mobile phase A: 200 mM hexafluoroisopropanol+ 8.15 mM triethylamine pH7.9
Mobile phase B: methanol
Flow rate: 0.3mL/min
Gradient:

| Time (min) | B% |
|---|---|
| 0 | 5 |
| 1 | 5 |
| 13 | 25 |
| 17 | 40 |
| 25 | 5 |

MS Equipment: Waters SQ Detector 2
Ion source: ESI
Ionization mode: ES⁻
M/Z: 600~3000

### Example 1

### Brief description of the synthesis pathway of the cap analogs

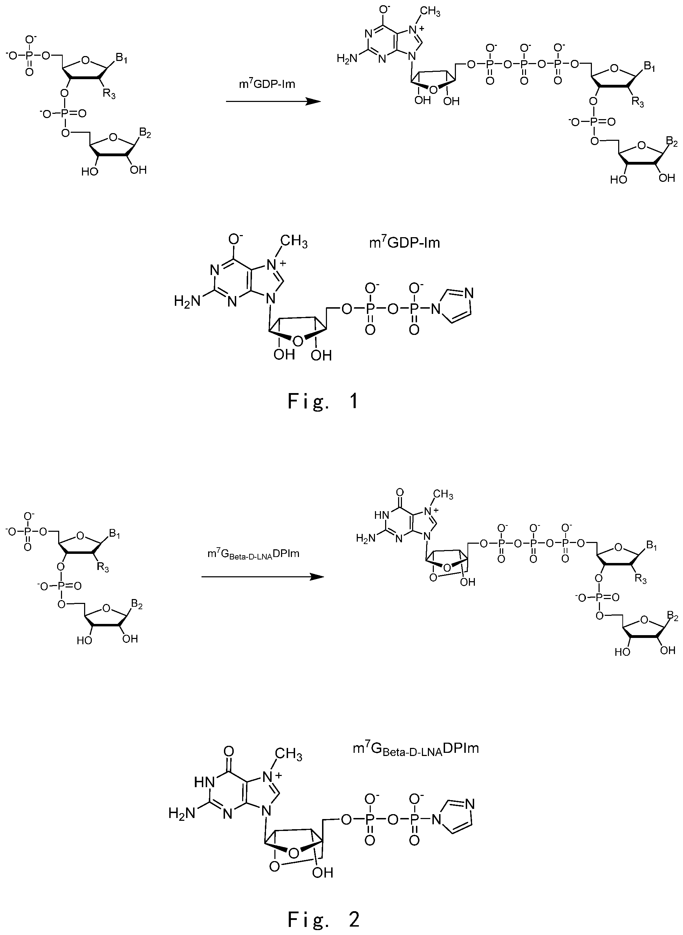

Synthesis of the cap analogs: raw materials 5'-O-DMT-2'-O-TBDMS phosphoramidite (rA^{Ac}, rC^{Ac}, rG^{dmf}, U) and 2'-O-MOE-3'-O-phosphoramidite (A_{2'O-MOE}^{Ac}, C_{2'O-MOE}^{Ac} , G_{2'O-MOE}^{dmf} U_{2'O-MOE},) and 2'-O-Ethyl-3'-O-phosphoramidite (A_{2'O-ethyl} ^{Ac}, C_{2'O-ethyl} ^{Ac} , G_{2'O-ethyl} ^{dmf} , U_{2'O-ethyl}) were purchased from Hongene, Bis-cyanoethyl-N,N-diisopropyl CED phosphoramidite was from ChemGenes. Conventional solid-phase synthesis procedure in the art (Bioautomation Mermade 12 solid phase synthesizer) was used to synthesize pNpN dinucleotide, which was then reacted with phosphor-site imidazolium salt of 7me-2'-O-4'-C-Locked-Guanosine 5'-diphosphate (m⁷G_{Beta-D-LNA}DPIm), or 2'-O,4'-C-methylene-a-L-ribofuranosyl Guanosine 5'-diphosphate (^{m7}G_{Alpha-L-LNA}DPIm), or N7-methylguanosine 5'-diphosphate(^{m7}GDPIm) to produce an initial cap oligonucleotide composition. Purified product was produced using DEAE-650s (Toyopearl) and semi-preparation RT-HPLC column. Synthesis procedure can be found in Figs. 1~3.

### Example 2

### Synthesis of mRNAs comprising cap analog

### (1) Preparation of template DNA

The template is usually formed of linear plasmid which comprises a target sequence inserted at the multiple cloning site and a promoter for the corresponding polymerase upstream from the target sequence, such as T7, T3, or SP6 promoter. The plasmid needs to be linearized before use. DNA can be linearized via digestion using suitable restriction endonucleases, and then purified by, for example, phenol chloroform treatment followed by ethanol precipitation. The restriction endonucleases used for the linearization of plasmid should be able to produce blunt end or 5'-overhang (linearizing template with an enzyme capable of producing 3'-overhang leads to abnormal transcript).

### (2) Preparation of mixed enzymes

| Mixed enzymes | Final concentration |
|---|---|
| T7 RNA polymerase | 100 U/ul |
| Inorganic pyrophosphatase | 0.1 U/ul |
| nuclease inhibitor | 20 U/ul |

### (3) Preparation of 10 × reaction buffer

| 10 × reaction buffer | Final concentration |
|---|---|
| HEPES-NaOH pH7.5 @ 25 °C | 400 mM |
| MgCl₂ | 200 mM |
| Spermidine | 10 mM |
| Dithiothreitol | 100 mM |

### (4) T7 in vitro transcription reaction

| | |
|---|---|
| 10xreaction buffer | 2 ul |
| 100mM initial cap oligonucleotide | 2 ul |
| 100mM ATP | 1 ul |
| 100mM GTP | 0.5 ul |
| 100mM CTP | 1 ul |
| 100mM N1-Me-ψUTP | 1 ul |
| Mixed enzymes | 2 ul |
| Template | 1 ul |
| H₂O (without nuclease pollution) | 9 ul |
| Total volume | 20 ul |

Cap analogs are ^{m7}GpppApG, ^{m7}G pppA_{2'Ome} pG, ^{m7}G_{3'Ome}pppA_{2'O-ethyl}pG, ^{m7}G_{2'Ome}pppA_{2'O-ethyl}pG, ^{m7}G_{3'Ome}pppA_{2'O-MOE}pG, ^{m7}G_{2'Ome}pppA_{2'O-MOE}pG, ^{m7}Gppp(N6-methyladenine)_{2'O-MOE}pG), ^{m7}G_{2'Ome}pppC_{2'O-ethyl}pC, ^{m7}G_{3'Ome}pppA_{2'O-MOE}pU, respectively.
reacting at 37 °C for 2hs,
adding DNase I (1U), and reacting at 37 °C for 30mins.

### (5) Treatment with Antarctic phosphatase:

The reaction solution was purified with Thermo MEGAclear^{™} Transcription Clean-Up Kit. To the purified mRNA, 2ul 5U/ul Antarctic phosphatase was added and mixed for reaction at 37 °C for 1h.

After treatment, the yield of mRNA was analyzed using spectrophotometer. The results of the yield are shown in Table 1.

### (6) Purification of the reaction solution

Purification was carried out by HPLC under the following conditions: Buffer B (0.1M triethylamine-acetic acid pH7.0 and 25% acetonitrile), Buffer A (0.1M triethylamine-acetic acid pH7.0), gradient from 30% to 60% Buffer A, in about 30min, 1ml/min. The fractions comprising RNA were combined and precipitated by isopropanol, and quantified using spectrophotometer.

### (7) Determination of capping efficiency

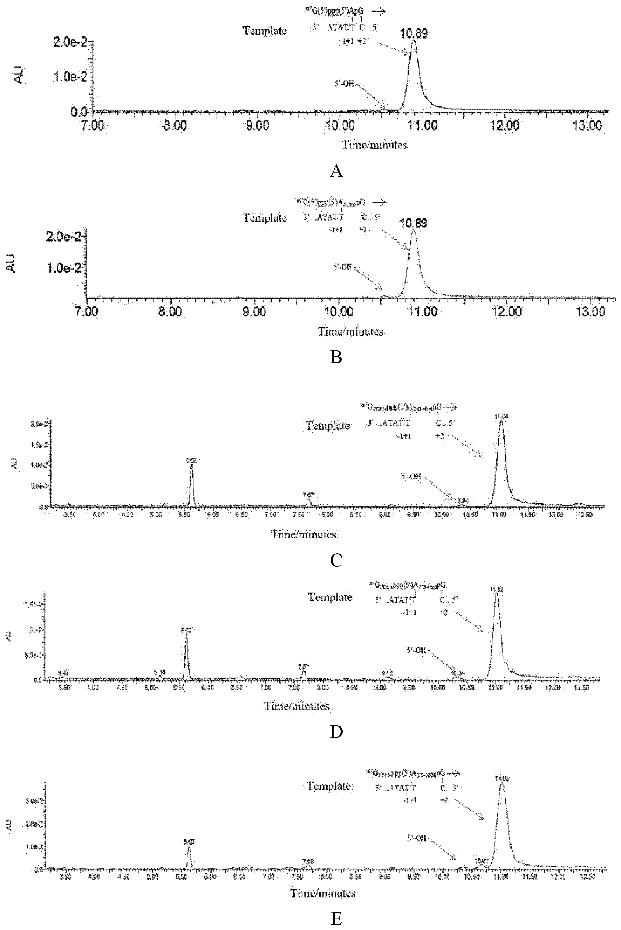

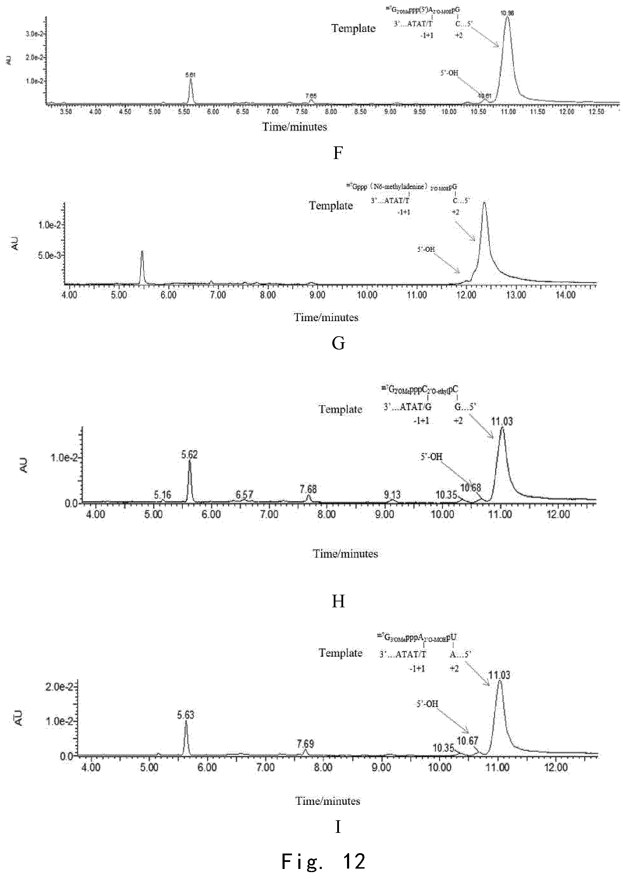

For each tested initial cap oligonucleotide, the ratio of capped mRNA was assessed by analyzing liquid chromatography-mass spectrometry (LC-MS) results. In the experiment, a DNA probe complementary to the mRNA was used. Upon the hybridization of the DNA to the RNA, RNaseH specifically hydrolyzed the phosphodiester bond in the RNA to produce small fragments of RNAs with homogeneous 3' end. The molecular weight and percentage of the small RNA fragments were determined by LC-MS, and were used to determine capping of the RNAs in the transcription reaction. Since the presence and absence of cap at 5' end can be distinguished by the molecule weight, the capping efficiency can be determined via the molecule weight and percentage of trunked 5' end-containing RNAs. In addition, from the calculation of the molecule weight, it can be determined that the caps added are the capping nucleotides prepared according to the synthesis pathway of Example 1. The result of capping efficiency can be found in Table 1 and Fig. 12.

### (8) Identification of protein expression

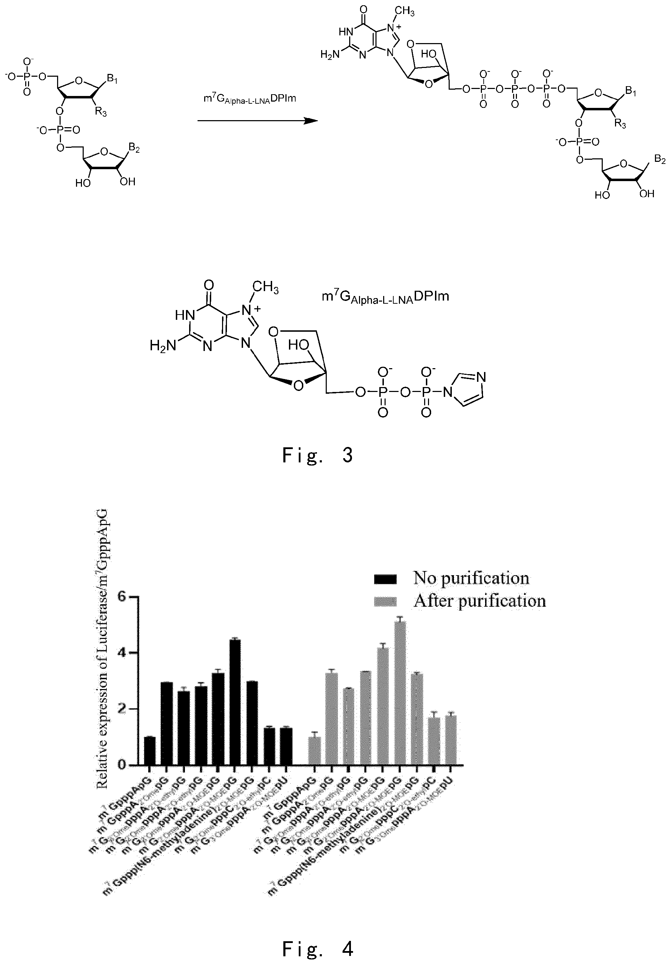

In this example, Hela cells (ATCC CCL-2) were transfected with luciferase mRNAs (Gaussia luciferase) comprising various initial cap structures (the initial cap nucleotide structures comprised are: ^{m7}GpppApG, ^{m7}GpppA_{2'Ome} pG, ^{m7}G_{3'Ome}pppA_{2'O-ethyl}pG, ^{m7}G_{2'Ome}pppA_{2'O-ethyl}pG, ^{m7}G_{3'Ome}pppA_{2'O-MOE}pG, ^{m7}G_{2'Ome}pppA_{2'O-MOE}pG, ^{m7}Gppp(N6-methyladenine)_{2'O-MOE}pG, ^{m7}G_{2'Ome}pppC_{2'O-ethyl}pC, ^{m7}G_{3'Ome}pppA_{2'O-MOE}pU), and the signal intensity of luciferase was detected by microplate reader after 24 h. The fluorescence signal intensity was proportional to the abundance of protein expression. Hela cells were seeded at a density of 4×10⁵/well in 6-well plates, and transfection was carried out when the cell density reached approximately 80%. Each well was transfected with 2 ug mRNA, and the transfection reagent was Lipofectamine MessengerMAX Transfection Reagent (Invitrogen), and the transfection steps were carried out according to the instructions. Twenty-four hours after transfection, the signal intensity of luciferase was measured on microplate reader. Measurement of luciferase fluorescence: the transfected cell culture solution was transferred to a 1.5 mL centrifuge tube and centrifuged, 10 ul of cell supernatant was taken and added to a 96-well plate, and 50 ul of 10ng/ml substrate was added, and then the fluorescence intensity was measured on Synergy HI (BioTek) instrument. The expression of each mRNA was required to be measured 6 times and the expression results were relative values to the results of ^{m7}GpppApG cap. Data are expressed as mean± standard deviation and the results obtained are shown in Table 1. Protein expression identification of unpurified mRNAs was also completed and the results are shown in Table 1 and Fig. 4.

**Table 1: Statistic data of mRNAs comprising various initial cap nucleotides**

| mRNA containing the following initial cap nucleotide | Yield of synthesis (20ul IVT system) | Capping efficiency | Relative Luciferase Unit RLU (after purification) | Relative Luciferase Unit RLU (no purification) |
|---|---|---|---|---|
| ^{m7}GpppApG | 50ug | 98% | 1±0.19 | 1±0.02 |
| ^{m7}GpppA_{2'Ome} pG | 52ug | 97% | 3.3±0.13 | 2.94±0.02 |
| ^{m7}G_{3'Ome}pppA_{2'O-ethyl}pG | 45ug | 94% | 2.73±0.03 | 2.63±0.15 |
| ^{m7}G_{2'Ome}pppA_{2'O-ethyl}pG | 48ug | 92% | 3.34±0.01 | 2.83±0.11 |
| ^{m7}G_{3'Ome}pppA_{2'O-MOE}pG | 51ug | 89% | 4.18±0.16 | 3.3±0.13 |
| ^{m7}G_{2'Ome}pppA_{2'O-MOE}pG | 45ug | 88% | 5.11±0.17 | 4.47±0.07 |
| ^{m7}Gppp(N6-methyladenine) _{2'O-MOE}pG | 45ug | 84% | 3.23±0.07 | 2.98±0.02 |
| ^{m7}G_{2'Ome}pppC_{2'O-ethyl}pC | 48ug | 92% | 1.71±0.18 | 1.33±0.06 |
| ^{m7}G_{3'Ome}pppA_{2'O-MOE}pU | 48ug | 93% | 1.76±0.12 | 1.32±0.06 |

### (9) Determination of the binding ability of mRNA to RIG-I and IFIT1

In this example, Hela cells (ATCC CCL-2) were transfected with luciferase mRNAs (Gaussia luciferase) comprising various initial cap structures (the initial cap nucleotide structures comprised are: ^{m7}GpppApG, ^{m7}GpppA_{2'Ome} pG, ^{m7}G_{3'Ome}pppA_{2'O-ethyl}pG, ^{m7}G_{2'Ome}pppA_{2'O-ethyl}pG, ^{m7}G_{3'Ome}pppA_{2'O-MOE}pG, ^{m7}G_{2'Ome}pppA_{2'O-MOE}pG, ^{m7}Gppp(N6-methyladenine)_{2'O-MOE}pG, ^{m7}G_{2'Ome}pppC_{2'O-ethyl}pC, ^{m7}G_{3'Ome}pppA_{2'O-MOE}pU), and collected after 24 hours. The intracellular proteins RIG-I and IFIT1 were co-precipitated with the RNAs to which they bound by RNA co-immunoprecipation. The mRNAs were subjected to reverse transcription and real-time quantitative PCR, and the relative abundance of the mRNAs is proportional to their RIG-I and IFIT1 binding ability.

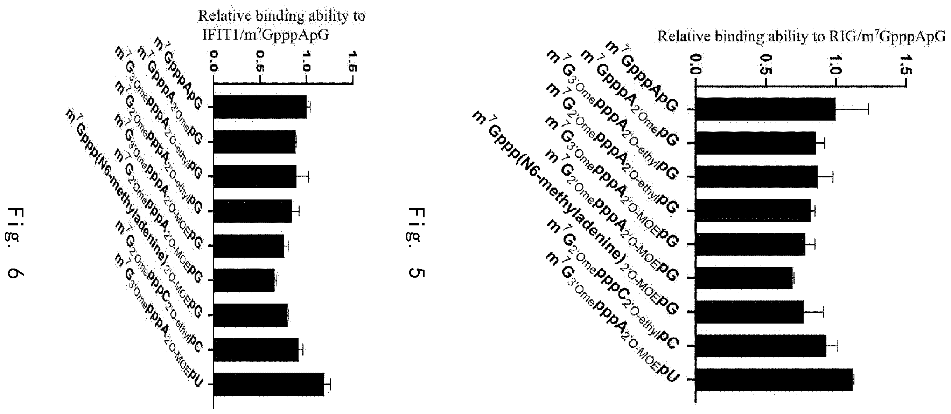

Hela cells were seeded at a cell density of 4×10⁵/well in 6-well plates, and transfected with mRNA at 2 ug mRNA/well when the cell density reached about 80% using Lipofectamine MessengerMAX Transfection Reagent (Invitrogen) according to the transfection steps in the instruction. The cells were collected after 24 hours, and incubated at RT for 10 minutes after the addition of fixative solution. The reaction was then terminated by adding glycine at a suitable concentration, and the cells were collected by centrifugation. Appropriate amount of lysis solution was added to the cells. The mixture was incubated on ice for 30 minutes and centrifuged to collect supernatant. Appropriate amount of (2-4 ng) anti-RIG-I and IFIT1 antibodies (Abcam) were added to the supernatant, and the mixture was incubated overnight at 4 °C on a shaker, and then 20 ul Protein A/G magnetic beads were added and incubated for 2 hours at 4 °C on a shaker. The beads were rinsed using magnetic grate for 5 minutes and repeated 3 times. After that, 1 mL TRIzol reagent was added to the magnetic beads to extract RNAs which were then reverse transcribed into cDNAs. The expression of related genes was detected using real-time quantitative fluorescent PCR with β-actin as the internal reference. The detection of each gene was repeated 3 times, and the result of the expression of each gene is expressed as the relative value to the result of ^{m7}GpppApG cap. Data is shown as mean ± SD, and the obtained results are shown in Table 2, and Figs. 5 and 6.

**Table 2. Statistical data of the binding ability of mRNAs with various initial cap nucleotides to RIG-I and IFIT1**

| mRNA containing the following initial cap nucleotide | Relative binding ability to RIG | Relative binding ability to IFIT1 |
|---|---|---|
| ^{m7}GpppApG | 1±0.23 | 1±0.04 |
| ^{m7}GpppA_{2'Ome} pG | 0.86±0.06 | 0.88±0.01 |
| ^{m7}G_{3'Ome}pppA_{2'O-ethyl}pG | 0.87±0.11 | 0.89±0.13 |
| ^{m7}G_{2'Ome}pppA_{2'O-ethyl}pG | 0.82±0.03 | 0.84±0.08 |
| ^{m7}G_{3'Ome}pppA_{2'O-MOE}pG | 0.78±0.07 | 0.76±0.04 |
| ^{m7}G_{2'Ome}pppA_{2'O-MOE}pG | 0.69±0.01 | 0.66±0.02 |
| ^{m7}Gppp(N6-methyladenine)_{2'O-MOE}pG | 0.77±0.14 | 0.79±0.01 |
| ^{m7}G_{2'Ome}pppC_{2'O-ethyl}pC | 0.93±0.08 | 0.91±0.05 |
| ^{m7}G_{3'Ome}pppA_{2'O-MOE}pU | 1.12±0.01 | 1.19±0.07 |

### (10) Determination of immunogenicity

In this example, Hela cells (ATCC CCL-2) were transfected with luciferase mRNAs (Gaussia luciferase) comprising various initial cap structures (the initial cap nucleotide structures comprised are: ^{m7}GpppApG, ^{m7}GpppA_{2'Ome} pG, ^{m7}G_{3'Ome}pppA_{2'O-ethyl}pG, ^{m7}G_{2'Ome}pppA_{2'O-ethyl}pG, ^{m7}G_{3'Ome}pppA_{2'O-MOE}pG, ^{m7}G_{2'Ome}pppA_{2'O-MOE}pG, ^{m7}Gppp(N6-methyladenine)_{2'O-MOE}pG, ^{m7}G_{2'Ome}pppC_{2'O-ethyl}pC, ^{m7}G_{3'Ome}pppA_{2'O-MOE}pU), and collected after 24 hours. The expression of intracellular inflammatory factors was detected, and the relative abundance of the factors is in proportional to the immunogenicity of the mRNA.

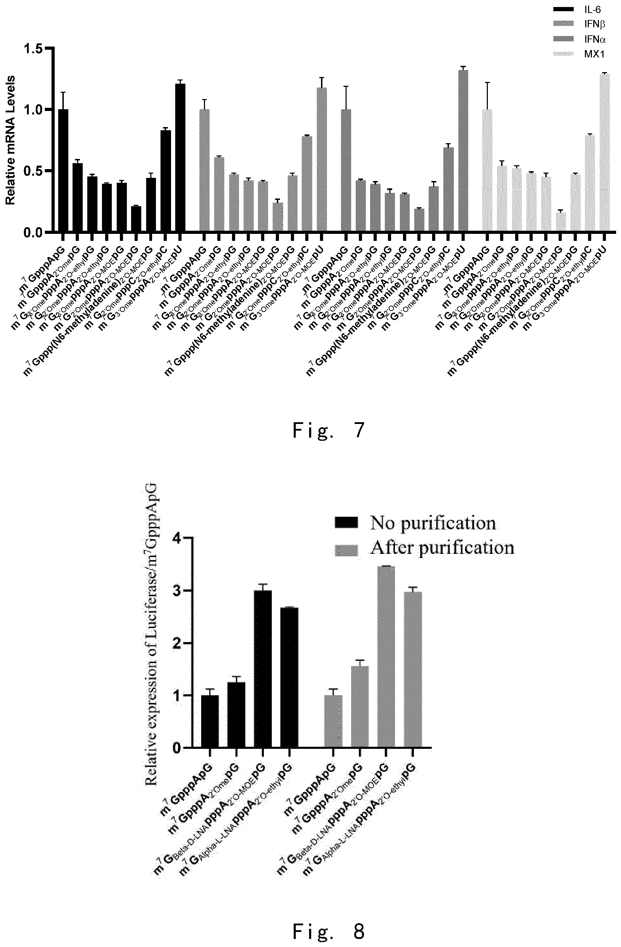

Hela cells were seeded at a density of 4×10⁵/well in 6-well plates, and transfection was carried out when the cell density reached approximately 80%. Each well was transfected with 2 ug mRNA, and the transfection reagent was Lipofectamine MessengerMAX Transfection Reagent (Invitrogen), and the transfection steps were carried out according to the instructions. Twenty-four hours after transfection, the cells were collected and TRIzol was used to extract RNAs which were then reverse transcribed into cDNAs. The expression of intracellular inflammatory factors was detected using real-time quantitative fluorescent PCR with β-actin as the internal reference. The detection of each gene was repeated 3 times, and the result of the expression of each gene is expressed as the relative value to the result of ^{m7}GpppApG cap. Data is shown as mean ± SD, and the obtained results are shown in Table 3, and Fig. 7.

**Table 3. Statistical data of the immunogenicity of mRNAs with various initial cap nucleotides**

| mRNA containing the following initial cap nucleotide | Expression of IL-6 | Expression of INFB | Expression of IFNA | Expression of MX1 |
|---|---|---|---|---|
| ^{m7}GpppApG | 1±0.14 | 1±0.08 | 1±0.19 | 1±0.22 |
| ^{m7}GpppA_{2'Ome}pG | 0.56±0.03 | 0.61±0.01 | 0.42±0.01 | 0.54±0.04 |
| ^{m7}G_{3'Ome}pppA_{2'O-ethyl}pG | 0.45±0.02 | 0.47±0.01 | 0.39±0.02 | 0.52±0.02 |
| ^{m7}G_{2'Ome}pppA_{2'O-ethyl}pG | 0.39±0.01 | 0.42±0.02 | 0.32±0.03 | 0.48±0.01 |
| ^{m7}G_{3'Ome}pppA_{2'O-MOE}pG | 0.40±0.02 | 0.41±0.01 | 0.31±0.01 | 0.45±0.03 |
| ^{m7}G_{2'Ome}pppA_{2'O-MOE}pG | 0.21±0.01 | 0.24±0.03 | 0.19±0.01 | 0.16±0.02 |
| ^{m7}Gppp(N6-methyladenine) _{2'O-MOE}pG | 0.44±0.04 | 0.46±0.02 | 0.37±0.04 | 0.47±0.01 |
| ^{m7}G_{2'Ome}pppC_{2'O-ethyl}pC | 0.83±0.02 | 0.78±0.01 | 0.69±0.03 | 0.79±0.01 |
| ^{m7}G_{3'Ome}pppA_{2'O-MOE}pU | 1.21±0.03 | 1.18±0.08 | 1.32±0.03 | 1.29±0.01 |

### Example 3

### Preparation of mRNAs comprising cap analogs ^{m7}G_{Beta-D-LNA}pppA_{2'O}-_{MOE}pG and ^{m7}G_{Alpha-L-LNA}pppA_{2'O-ethyl} pG and expressions thereof

### (1) Preparation of template DNA

The template is usually formed of linear plasmid which comprises a target sequence inserted at the multiple cloning site and a promoter for the corresponding polymerase upstream from the target sequence, such as T7, T3, or SP6 promoter. The plasmid needs to be linearized before use. DNA can be linearized via digestion using suitable restriction endonucleases, and then purified by suitable methods, for example, phenol chloroform treatment followed by ethanol precipitation. The restriction endonucleases used for the linearization of plasmid should be able to produce blunt end or 5'-overhang (linearizing template with an enzyme capable of producing 3'-overhang leads to abnormal transcript).

### (2) Preparation of mixed enzymes

| Mixed enzymes | Final concentration |
|---|---|
| Thermostable T7 RNA polymerase (NEB) | 100U/ul |
| Thermostable inorganic pyrophosphatase (NEB) | 0.1U/ul |
| nuclease inhibitor | 20U/ul |

### (3) Preparation of 10×reaction buffer

| 10xreaction buffer | Final concentration |
|---|---|
| HEPES-NaOH pH7.5@25°C | 400mM |
| MgCl₂ | 200mM |
| Spermidine | 10mM |
| Dithiothreitol | 100mM |

### (4) T7 in vitro transcription reaction

| | |
|---|---|
| 10×reaction buffer | 2ul |
| 100mM initial cap oligonucleotide | 2ul |
| 100mM ATP | 1ul |
| 100mM GTP | 0.5ul |
| 100mM CTP | 1ul |
| 100mM N1-Me-ψUTP | 1ul |
| Mixed enzymes | 2ul |
| Template | 1ul |
| H₂O (without nuclease pollution) | 9ul |
| Total volume | 20ul |

Initial cap oligonucleotides are ^{m7}GpppApG, ^{m7}G pppA_{2'Ome} pG (control), ^{m7}G_{Beta-D-LNA}pppA_{2'O-MOE}pG, and ^{m7}G_{Alpha-L-LNA}pppA_{2'O-ethyl} pG, respectively.
reacting at 50 °C for 2hs,
adding DNase I (1U), and reacting at 37 °C for 30mins.

### (5) Treatment with Antarctic phosphatase:

The reaction solution was purified with Thermo MEGAclear^{™} Transcription Clean-Up Kit. To the purified mRNA, 2ul 5U/ul Antarctic phosphatase was added and mixed for reaction at 37 °C for 1h.

After treatment, the yield of mRNA was analyzed using spectrophotometer. The results of the yield are shown in Table 4.

### (6) Purification of the reaction solution

Purification was carried out by HPLC under the following conditions: Buffer B (0.1M triethylamine-acetic acid pH7.0 and 25% acetonitrile), Buffer A (0.1M triethylamine-acetic acid pH7.0), gradient from 30% to 60% Buffer A, in about 30min, 1ml/min. The fractions comprising RNA were combined and precipitated by isopropanol, and quantified using spectrophotometer.

### (7) Determination of capping efficiency

For each tested initial cap oligonucleotide, the ratio of capped mRNA was assessed by analyzing liquid chromatography-mass spectrometry (LC-MS) results. In the experiment, a DNA probe complementary to the mRNA was used. Upon the hybridization of the DNA to the RNA, RNaseH specifically hydrolyzed the phosphodiester bond in the RNA to produce small fragments of RNAs with homogeneous 3' end. The molecular weight and percentage of the small RNA fragments were determined by LC-MS, and were used to determine capping of the RNAs in the transcription reaction. Since the presence and absence of cap at 5' end can be distinguished by the molecule weight, the capping efficiency can be determined via the molecule weight and percentage of trunked 5' end-containing RNAs. In addition, from the calculation of the molecule weight, it can be determined that the caps added are the capping nucleotides. The result of capping efficiency can be found in Table 4 and Fig. 13.

### (8) Identification of protein expression

In this example, Hela cells (ATCC CCL-2) were transfected with luciferase mRNAs (Gaussia luciferase) comprising various initial cap structures (the initial cap nucleotide structures comprised are: ^{m7}GpppApG, ^{m7}G pppA_{2'Ome} pG, ^{m7}G_{Beta-D-LNA}pppA_{2'O-MOE}pG and ^{m7}G_{Alpha-L-LNA}pppA_{2'O-ethyl} pG), and the signal intensity of luciferase was detected by microplate reader after 24 h. The fluorescence signal intensity was proportional to the abundance of protein expression.

Hela cells were seeded at a density of 4×10⁵/well in 6-well plates, and transfection was carried out when the cell density reached approximately 80%. Each well was transfected with 2 ug mRNA, and the transfection reagent was Lipofectamine MessengerMAX Transfection Reagent (Invitrogen), and the transfection steps were carried out according to the instructions. Twenty-four hours after transfection, the signal intensity of luciferase was measured on microplate reader. Measurement of luciferase fluorescence: the transfected cell culture solution was transferred to a 1.5 mL centrifuge tube and centrifuged, 10 ul of cell supernatant was taken and added to a 96-well plate, and 50 ul of 10ng/ml substrate was added, and then the fluorescence intensity was measured on Synergy HI (BioTek) instrument. The expression of each mRNA was required to be measured 6 times and the expression results were relative values to the results of ^{m7}GpppApG cap. Data are expressed as mean± standard deviation and the results obtained are shown in Table 4. Protein expression identification of unpurified mRNAs was also completed and the results are shown in Table 4 and Fig. 8.

**Table 4. Statistic data of mRNAs comprising initial cap nucleotides ^{m7}G_{Beta-D-LNA}pppA_{2'O-MOE}pG and ^{m7}G_{Alpha-L-LNA}pppA_{2'O-ethyl} pG**

| mRNA containing the following initial cap nucleotide | Yield of synthesis (20ul IVT system) | Capping efficiency | Relative Luciferase Unit RLU (after purification) | Relative Luciferase Unit RLU (no purification) |
|---|---|---|---|---|
| ^{m7}GpppApG | 50ug | 93% | 1±0.12 | 1±0.02 |
| ^{m7}GpppA_{2'Ome} pG | 52ug | 95% | 1.56±0.11 | 1.25±0.11 |
| ^{m7}G_{Beta-D-LNA}pppA_{2'O-MOE}pG | 40ug | 70% | 3.45±0.01 | 3±0.12 |
| ^{m7}G_{Alpha-L-LNA}pppA_{2'O-ethyl} pG | 38ug | 63% | 2.97±0.09 | 2.67±0.02 |

### (9) Determination of the binding ability of mRNA to RIG-I and IFIT1

In this example, Hela cells (ATCC CCL-2) were transfected with luciferase mRNAs (Gaussia luciferase) comprising various initial cap structures (the initial cap nucleotide structures comprised are: ^{m7}GpppApG, ^{m7}G pppA_{2'Ome} pG, ^{m7}G_{Beta-D-LNA}pppA_{2'O-MOE}pG and ^{m7}G_{Alpha-L-LNA}pppA_{2'O-ethyl} pG), and collected after 24 hours. The intracellular proteins RIG-I and IFIT1 were co-precipitated with the RNAs to which they bound by RNA co-immunoprecipitation. The mRNAs were subjected to reverse transcription and real-time quantitative PCR, and the relative abundance of the mRNAs is proportional to their RIG-I and IFIT1 binding ability.

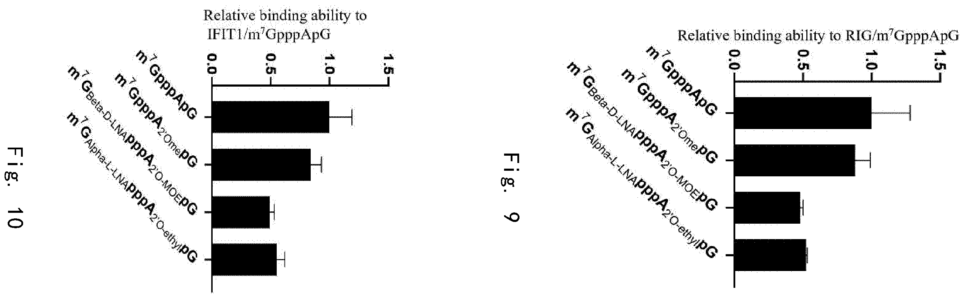

Hela cells were seeded at a cell density of 4×10⁵/well in 6-well plates, and transfected with mRNA at 2 ug mRNA/well when the cell density reached about 80% using Lipofectamine MessengerMAX Transfection Reagent (Invitrogen) as the transfection reagent according to the transfection steps in the instruction. The cells were collected after 24 hours, and incubated at RT for 10 minutes after the addition of fixative solution. The reaction was terminated by adding glycine at a suitable concentration, and the cells were collected by centrifugation. Appropriate amount of lysis solution was added to the cells. The mixture was incubated on ice for 30 minutes and centrifuged to collect supernatant. Appropriate amount of (2-4 ng) anti-RIG-I and IFIT1 antibodies (Abcam) were added to the supernatant, and the mixture was incubated overnight at 4 °C on a shaker, and then 20 ul Protein A/G magnetic beads were added and incubated for 2 hours at 4 °C on a shaker. The beads were rinsed using magnetic grate for 5 minutes and repeated 3 times. After that, 1 mL TRIzol reagent was added to the magnetic beads to extract RNAs which were then reverse transcribed into cDNAs. The expression of related genes was detected using real-time quantitative fluorescent PCR with β-actin as the internal reference. The detection of each gene was repeated 3 times, and the result of the expression of each gene is expressed as the relative value to the result of ^{m7}GpppApG cap. Data are shown as mean ± SD, and the obtained results are shown in Table 5, and Figs. 9 and 10.

**Table 5. Statistical data of the binding ability of mRNAs with initial cap nucleotides ^{m7}G_{Beta-D-LNA}pppA_{2'O-MOE}pG and ^{m7}G_{Alpha-L-LNA}pppA_{2'O-ethyl} pG to RIG-I and IFIT 1**

| mRNA containing the following initial cap nucleotide | Relative binding ability to RIG | Relative binding ability to IFIT1 |
|---|---|---|
| ^{m7}GpppApG | 1±0.28 | 1±0.19 |
| ^{m7}GpppA_{2'Ome} pG | 0.88±0.11 | 0.84±0.09 |
| ^{m7}G_{Beta-D-LNA}pppA_{2'O-MOE}pG | 0.48±0.02 | 0.49±0.04 |
| ^{m7}G_{Alpha-L-LNA}pppA_{2'O-ethyl} pG | 0.52±0.01 | 0.55±0.07 |

### (10) Determination of immunogenicity

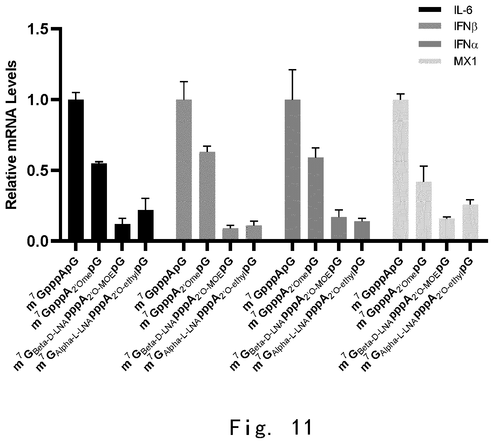

In this example, Hela cells (ATCC CCL-2) were transfected with luciferase mRNAs (Gaussia luciferase) comprising various initial cap structures (the initial cap nucleotide structures comprised are: ^{m7}GpppApG, ^{m7}G pppA_{2'Ome} pG, ^{m7}G_{Beta-D-LNA}pppA_{2'O-MOE}pG, and ^{m7}G_{Alpha-L-LNA}pppA_{2'O-ethyl} pG), and collected after 24 hours. The expression of intracellular inflammatory factors was detected, and the relative abundant of the factors is in proportional to the immunogenicity of the mRNA. Hela cells were seeded at a density of 4×10⁵/well in 6-well plates, and transfection was carried out when the cell density reached approximately 80%. Each well was transfected with 2 ug mRNA, and the transfection reagent was Lipofectamine MessengerMAX Transfection Reagent (Invitrogen), and the transfection steps were carried out according to the instructions. Twenty-four hours after transfection, the cells were collected and TRIzol was used to extract RNAs which were then reverse transcribed into cDNAs. The expression of intracellular inflammatory factors was detected using real-time quantitative fluorescent PCR with β-actin as the internal reference. The detection of each gene was repeated 3 times, and the result of the expression of each gene is expressed as the relative value to the result of ^{m7}GpppApG cap. Data are shown as mean ± SD, and the obtained results are shown in Table 6, and Fig. 11.

**Table 6. Statistical data of the immunogenicity of mRNAs with initial cap nucleotides ^{m7}G_{Beta-D-LNA}pppA_{2'O-MOE}pG and ^{m7}G_{Alpha-L-LNA}pppA_{2'O-ethyl} pG**

| mRNA containing the following initial cap nucleotide | Expression of IL-6 | Expression of INFB | Expression of IFNA | Expression of Mx1 |
|---|---|---|---|---|
| ^{m7}GpppApG | 1±0.05 | 1±0.13 | 1±0.21 | 1±0.04 |
| ^{m7}GpppA_{2'Ome} pG | 0.55±0.01 | 0.63±0.04 | 0.59±0.07 | 0.42±0.11 |
| ^{m7}G_{Beta-D-LNA}pppA_{2'O-MOE}pG | 0.12±0.04 | 0.09±0.02 | 0.17±0.05 | 0.16±0.01 |
| ^{m7}G_{Alpha-L-LNA}pppA_{2'O-ethyl} pG | 0.22±0.08 | 0.11±0.03 | 0.14±0.02 | 0.26±0.03 |

The above are merely exemplary examples of the present invention, which are not intended to limit the scope of the substantive technical contents of the present invention. The substantive technical content of the present invention is broadly defined within the scope of the attached claims. Any technical entity or method completed by others, if completely identical to the scope of the attached claims or is an equivalent change, will be deemed to be covered by the scope of the claims.

## Claims

1. A cap analog of formula (I): 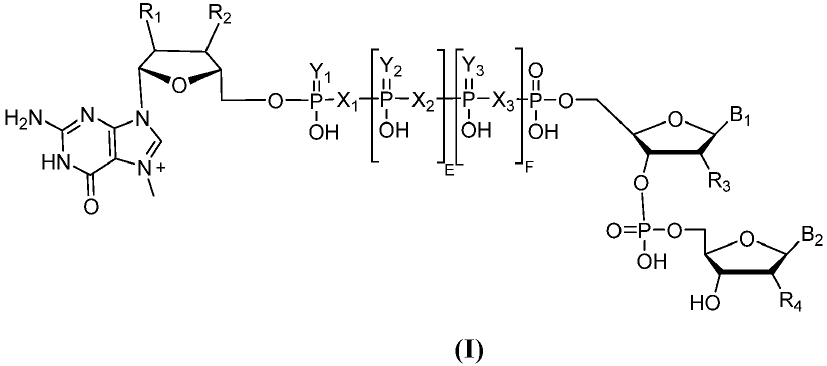
wherein, in the formula, B₁ and B₂ are independently natural or modified base;
E and F are independently 0 or 1;
R₁ is H, OH, alkyl, O-alkyl, halo, or O, wherein said O taken together with carbon atoms at 3' position and 5' position form a bridged ring;
R₂ is H, OH, alkyl, O-alkyl, or halo;
R₃ is O-R₅-R₆;
R₄ is H, OH, O-methyl or O-R₅-R₆;
R₅ is substituted or unsubstituted C₁₋₂₀ alkyl;
R₆ is substituted or unsubstituted O-alkyl, substituted or unsubstituted S-alkyl, substituted or unsubstituted NH-alkyl, substituted or unsubstituted N-dialkyl, substituted or unsubstituted O-aryl, substituted or unsubstituted S-aryl, substituted or unsubstituted NH-aryl, substituted or unsubstituted O-aralkyl, substituted or unsubstituted S-aralkyl, substituted or unsubstituted NH-aralkyl, or H (when R₅ is substituted or unsubstituted C₂₋₂₀ alkyl);
X₁, X₂, and X₃ are independently O, CH₂, or NH;
Y₁, Y₂, and Y₃ are independently O, S, Se, or BH₃.

2. The cap analog according to claim 1, wherein R₃ is OCH₂CH₃, OCH₂OCH₃, or OCH₂CH₂OCH₃.

3. The cap analog according to claim 1, wherein R₄ is OH, OCH₂CH₃, OCH₂OCH₃, or OCH₂CH₂OCH₃.

4. The cap analog according to claim 1, wherein B₁ and B₂ are independently adenine, N6-methyladenine, guanine, uracil, or thymine.

5. The cap analog according to claim 1, wherein said cap analog is selected from: ^{m7}GpppA_{2'O-ethyl}pG, ^{m7}GpppA_{2'O-ethyl}pA, ^{m7}GpppA_{2'O-ethyl}pC, ^{m7}GpppA_{2'O-ethyl}pU, ^{m7}GpppC_{2'O-ethyl}pA, ^{m7}GpppC_{2'O-ethyl}pG, ^{m7}GpppC_{2'O-ethyl}pC, ^{m7}GpppC_{2'O-ethyl}pU, ^{m7}GpppG_{2'O-ethyl}pA, ^{m7}GpppG_{2'O-ethyl}pC, ^{m7}GpppG_{2'O-ethyl}pG, ^{m7}GpppG_{2'O-ethyl}pU, ^{m7}GpppU_{2'O-ethyl}pA, ^{m7}GpppU_{2'O-ethyl}pC, ^{m7}GpppU_{2'O-ethyl}pC, or ^{m7}GpppU_{2'O-ethyl}pU.

6. The cap analog according to claim 1, wherein said cap analog is selected from: ^{m7}G_{3'Ome}pppA_{2'O-ethyl}pG, ^{m7}G_{3'Ome} pppA_{2'O-ethyl}pA, ^{m7}G_{3'Ome} pppA_{2'O-ethyl}pC, ^{m7}G_{3'Ome}pppA_{2'O-ethyl}pU, ^{m7}G_{3'Ome}pppC_{2'O-ethyl}pA, ^{m7}G_{3'Ome}pppC_{2'O-ethyl}pG, ^{m7}G_{3'Ome}pppC_{2'O-ethyl}pC, ^{m7}G_{3'Ome}pppC_{2'O-ethyl}pU, ^{m7}G_{3'Ome}pppG_{2'O-ethyl}pA, ^{m7}G_{3'Ome}pppG_{2'O-ethyl}pC, ^{m7}G_{3'Ome}pppG_{2'O-ethyl}pG, ^{m7}G_{3'Ome}pppG_{2'O-ethyl}pU, ^{m7}G_{3'Ome}pppU_{2'O-ethyl}pA, ^{m7}G_{3'Ome}pppU_{2'O-ethyl}pC, ^{m7}G_{3'Ome}pppU_{2'O-ethyl}pC, or ^{m7}G_{3'Ome}pppU_{2'O-ethyl}pU.

7. The cap analog according to claim 1, wherein said cap analog is selected from: ^{m7}G_{2'Ome}pppA_{2'O-ethyl}pG, ^{m7}G_{2'Ome}pppA_{2'O-ethyl}pA, ^{m7}G_{2'ome}pppA_{2'o-ethyl}pC, ^{m7}G_{2'Ome}pppA_{2'O-ethyl}pU, ^{m7}G_{2'Ome}pppC_{2'O-ethyl}pA, ^{m7}G_{2'Ome}pppC_{2'O-ethyl}pG, ^{m7}G_{2'Ome}pppC_{2'O-ethyl}pC, ^{m7}G_{2'Ome}pppC_{2'O-ethyl}pU, ^{m7}G_{2'Ome}pppG_{2'O-ethyl}pA, ^{m7}G_{2'Ome}pppG_{2'O-ethyl}pC, ^{m7}G_{2'Ome}pppG_{2'O-ethyl}pG, ^{m7}G_{2'Ome}pppG_{2'O-ethyl}pU, ^{m7}G_{2'Ome}pppU_{2'O-ethyl}pA, ^{m7}G_{2'Ome}pppU_{2'O-ethyl}pC, ^{m7}G_{2'Ome}pppU_{2'O-ethyl}pC, or ^{m7}G_{2'Ome}pppU_{2'O-ethyl}pU.

8. The cap analog according to claim 1, wherein said cap analog is selected from: ^{m7}Gppp(N6-methyladenine)_{2'O-ethyl}pG, ^{m7}Gppp(N6-methyladenine)_{2'O-ethyl}pA, ^{m7}Gppp(N6-methyladenine)_{2'O-ethyl}pC, ^{m7}Gppp(N6-methyladenine)_{2'O-ethyl}pU, ^{m7}G_{2'Ome}ppp(N6-methyladenine)_{2'O-ethyl}pA, ^{m7}G_{2'Ome}ppp(N6-methyladenine)_{2'O-ethyl}pG, ^{m7}G_{2'Ome}ppp(N6-methyladenine)_{2'O-ethyl}pC, ^{m7}G_{2'Ome}ppp(N6-methyladenine)_{2'O-ethyl}pU, ^{m7}G_{3'Ome}ppp(N6-methyladenine)_{2'O-ethyl}pA, ^{m7}G_{3'Ome}ppp(N6-methyladenine)_{2'O-ethyl}pC, ^{m7}G_{3'Ome}ppp(N6-methyladenine)_{2'O-ethyl}pC, or ^{m7}G_{3'Ome}ppp(N6-methyladenine)_{2'O-ethyl}pU.

9. The cap analog according to claim 1, wherein said cap analog is selected from: ^{m7}GpppA_{2'O-MOE}pG, ^{m7}GpppA_{2'O-MOE}pA, ^{m7}GpppA_{2'O-MOE}pC, ^{m7}GpppA_{2'O-MOE}pU, ^{m7}GpppC_{2'O-MOE}pA, ^{m7}GpppC_{2'O-MOE}pG, ^{m7}GpppC_{2'O-MOE}pC, ^{m7}GpppC_{2'O-MOE}pU, ^{m7}GpppG_{2'O-MOE}pA, ^{m7}GpppG_{2'O-MOE}pC, ^{m7}GpppG_{2'O-MOE}pG, ^{m7}GpppG_{2'O-MOE}pU, ^{m7}GpppU_{2'O-MOE}pA, ^{m7}GpppU_{2'O-MOE}pC, ^{m7}GpppU_{2'O-MOE}pC, or ^{m7}GpppU_{2'O-MOE}pU.

10. The cap analog according to claim 1, wherein said cap analog is selected from: ^{m7}G_{3'Ome}pppA_{2'O-MOE}pG, ^{m7}G_{3'Ome} pppA_{2'O-MOE}pA, ^{m7}G_{3'Ome}pppA_{2'O-MOE}pC, ^{m7}G_{3'Ome}pppA_{2'O-MOE}pU, ^{m7}G_{3'Ome}pppC_{2'O-MOE}pA, ^{m7}G_{3'Ome}pppC_{2'O-MOE}pG, ^{m7}G_{3'Ome}pppC_{2'O-MOE}pC, ^{m7}G_{3'Ome}pppC_{2'O-MOE}pU, ^{m7}G_{3'Ome}pppG_{2O-'MOE}pA, ^{m7}G_{3'Ome}pppG_{2'O-MOE}pC, ^{m7}G_{3'Ome}pppG_{2'O-MOE}pG, ^{m7}G_{3'Ome}pppG_{2'O-MOE}pU, ^{m7}G_{3'Ome}pppU_{2'O-MOE}pA, ^{m7}G_{3'Ome}pppU_{2'O-MOE}pC, ^{m7}G_{3'Ome}pppU_{2'O-MOE}pC, or ^{m7}G_{3'Ome}pppU_{2'O-MOE}pU.

11. The cap analog according to claim 1, wherein said cap analog is selected from: ^{m7}G_{2'Ome}pppA_{2'O-MOE}pG, ^{m7}G_{2'Ome} pppA_{2'O-MOE}pA, ^{m7}G_{2'Ome} pppA_{2'O-MOE}pC, ^{m7}G_{2'Ome}pppA_{2'O-MOE}pU, ^{m7}G_{2'Ome}pppC_{2'O-MOE}pA, ^{m7}G_{2'Ome}pppC_{2'O-MOE}pG, ^{m7}G_{2'Ome}pppC_{2'O-MOE}pC, ^{m7}G_{2'Ome}pppC_{2'O-MOE}pU, ^{m7}G_{2'Ome}pppG_{2'O-MOE}pA, ^{m7}G_{2'Ome}pppG_{2'O-MOE}pC, ^{m7}G_{2'Ome}pppG_{2'O-MOE}pG, ^{m7}G_{2'Ome}pppG_{2'O-MOE}pU, ^{m7}G_{2'Ome}pppU_{2'O-MOE}pA, ^{m7}G_{2'Ome}pppU_{2'O-MOE}pC, ^{m7}G_{2'Ome}pppU_{2'O-MOE}pC, or ^{m7}G_{2'Ome}pppU_{2'O-MOE}pU.

12. The cap analog according to claim 1, wherein said cap analog is selected from: ^{m7}Gppp(N6-methyladenine)_{2'O-MOE}pG, ^{m7}Gppp(N6-methyladenine) _{2'O-MOE}pA, ^{m7}Gppp(N6-methyladenine)_{2'O-MOE}pC, ^{m7}Gppp(N6-methyladenine) _{2'O-MOE}pU, ^{m7}G_{2'Ome}ppp(N6-methyladenine)_{2'O-MOE}pA, ^{m7}G_{2'Ome}ppp(N6-methyladenine) _{2'O-MOE}pG, ^{m7}G_{2'Ome}ppp(N6-methyladenine)_{2'O-MOE}pC, ^{m7}G_{2'Ome}ppp(N6-methyladenine)_{2'O-MOE}pU, ^{m7}G_{3'Ome}ppp(N6-methyladenine)_{2'O-MOE}pA, ^{m7}G_{3'Ome}ppp(N6-methyladenine)_{2'O-MOE}pC, ^{m7}G_{3'Ome}ppp(N6-methyladenine) _{2'O-MOE}pC, or ^{m7}G_{3'Ome}ppp(N6-methyladenine) _{2'O-MOE}pU.

13. The cap analog according to claim 1, wherein the structure of said cap analog is as shown in formula (I-a), or formula (I-b): 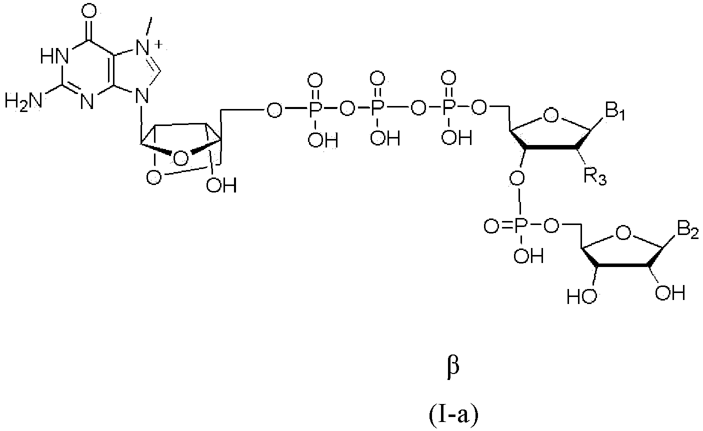 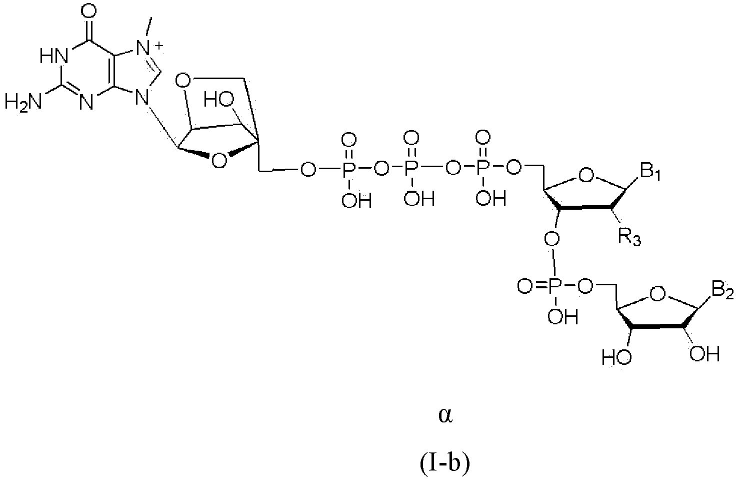 Wherein, in the formula, B₁ and B₂ are independently natural or modified base; R₃ is O-R₅-R₆.

14. The cap analog according to claim 13, wherein R₃ is OCH₂CH₃, OCH₂OCH₃, or OCH₂CH₂OCH₃.

15. The cap analog according to claim 13, wherein B₁ and B₂ are independently adenine, N6-methyladenine, guanine, uracil, or thymine.

16. A polynucleotide encoding a polypeptide of interest, wherein said polynucleotide comprises:
(a) at least one ORF region;
(b) at least one Kozak sequence of 5'UTR;
(c) 3'UTR; and
(d) at least one cap analog according to any one of claims 1-15 initially capped at the 5' end.

17. A pharmaceutical composition comprising the polynucleotide according to claim 16 and a pharmaceutically acceptable carrier.

18. The pharmaceutical composition according to claim 17, wherein said carrier is selected from: lipid nanoparticles (LNPs), liposomes, polymeric nanoparticles, solid lipid nanoparticles, or emulsions.

19. A method for preparing the polynucleotide according to claim 16, wherein the method comprises the steps of:
(1) preparing a DNA template;
(2) producing the polynucleotide according to claim 16 via *in vitro* transcription, wherein the reaction system comprises RNA polymerase, nucleoside triphosphate, and the cap analog according to any one of claims 1-15.

20. The method of claim 19, wherein the RNA polymerase is a phage-derived RNA polymerase.
